# EUROPÄISCHE PATENTSCHRIFT

(11) **EP 3 221 043 B1**
(45) Veröffentlichungstag und Bekanntmachung des Hinweises auf die Patenterteilung: **08.05.2019**
(21) Anmeldenummer: 15798006.1
(22) Anmeldetag: 17.11.2015
(51) Int. Cl.: B01J 19/00, C07C 201/08, C07C 205/06

(54) **VERFAHREN ZUR KONTINUIERLICHEN HERSTELLUNG EINES PRODUKTS ÜBER ZUMINDEST ZWEI MITEINANDER GEKOPPELTE CHEMISCHE REAKTIONEN**
METHOD FOR THE CONTINUOUS MANUFACTURE OF A PRODUCT OVER AT LEAST TWO MUTUALLY COUPLED CHEMICAL REACTIONS
PROCÉDÉ DE FABRICATION CONTINUE D'UN PRODUIT PAR LE BIAIS D'AU MOINS DEUX RÉACTIONS CHIMIQUES COUPLÉES ENSEMBLE

(30) Priorität: 20.11.2014 EP 14194180
(43) Veröffentlichungstag der Anmeldung: 27.09.2017
(73) Patentinhaber: Covestro Deutschland AG, 51373 Leverkusen (DE)
(72) Erfinder: PENNEMANN, Bernd, 51467 Bergisch Gladbach (DE); DIETERICH, Erwin, 50769 Köln (DE); HAMACHER, Heinz-Josef, 50181 Bedburg (DE)
(74) Vertreter: Levpat
(86) Internationale Anmeldenummer: PCT/EP2015/076857
(87) Internationale Veröffentlichungsnummer: WO 2016/079138

(56) Entgegenhaltungen:
- EP-A1- 1 880 989
- EP-A2- 0 903 336
- WO-A1-2005/075407
- H. Hermann ET AL: "Industrial Nitration of Toluene to Dinitrotoluene" In: "Nitration", 24. April 1996 (1996-04-24), American Chemical Society, Washington, DC, XP055184416, ISBN: 978-0-84-121568-9 Bd. 623, Seiten 234-249, DOI: 10.1021/bk-1996-0623.ch021, in der Anmeldung erwähnt Seite 243 - Seite 247

## Beschreibung

Die Erfindung betrifft ein Verfahren zur kontinuierlichen Herstellung eines Produkts über zumindest zwei miteinander gekoppelte chemische Reaktionen.

Eine Vielzahl von chemischen Produkten wird industriell in kontinuierlich oder semikontinuierlich betriebenen Verfahren hergestellt. Dabei werden ein oder mehrere Eingangsstoffe in einer chemischen Reaktion zu anderen Stoffen weiterverarbeitet. Einer oder mehrere der so erzeugten Stoffe können wiederum Eingang finden in einer nachfolgenden zweiten chemischen Reaktion, in der schließlich das gewünschte Endprodukt hergestellt wird. Kontinuierlich bedeutet dabei, dass den chemischen Reaktionen ununterbrochen Stoffe zugeführt werden, und dass die chemischen Reaktionen ununterbrochen Reaktionsprodukte (im nachfolgenden Verfahren die Ausgangsstoffe oder die Zwischenstoffe) erzeugen. "Ununterbrochen" bezieht sich dabei auf die Zeiträume, in denen die Reaktion tatsächlich stattfindet und schließt nicht aus, dass eine Reaktion bspw. infolge eines Wartungsstillstands unterbrochen werden kann. Im Folgenden werden die dem Herstellungsverfahren zugeführten Substanzen als Eingangsstoffe, abgekürzt "E" bezeichnet. Die aus dem Ausgang der Anlage strömenden Produkte werden als Ausgangsstoffe, abgekürzt "A" bezeichnet. Die Zwischenprodukte werden abgekürzt mit "Z" bezeichnet.

Im Rahmen solcher geschachtelten Prozesse werden aufgrund der vorgegebenen Stöchiometrie der zugrundeliegenden Reaktionsgleichung zwangsläufig Koppelprodukte mitgebildet. In industriellen Verfahren ist man bestrebt, aus Gründen der Wirtschaftlichkeit und des Umweltschutzes anfallende Koppelprodukte so weit wie möglich wiederzuverwenden. Beispielsweise fällt in vielen chemischen Prozessen wie der Herstellung von Isocyanaten bei der Phosgenierung der korrespondierenden Aminverbindungen Chlorwasserstoff als Koppelprodukt an, welches beispielsweise nach Aufoxidation zu Chlor erneut eingesetzt werden kann.
Bei mehrstufigen Reaktionen können ebenfalls solche Koppelprodukte anfallen, wobei es Fälle gibt, bei denen das Koppelprodukt einer nachgelagerten Reaktion als Eingangsstoff der ersten chemischen Reaktion zugeführt werden kann, ggf. nach vorheriger Aufbereitung. Hierzu gehört die Herstellung von Dinitrotoluol durch eine erste chemische Reaktion, der Nitrierung von Toluol zum Nitrotoluol, gefolgt von einer zweiten chemischen Reaktion, der Nitrierung von Nitrotoluol zu Dinitrotoluol. Die Nitrierung erfolgt üblicherweise durch ein Gemisch aus Salpetersäure und Schwefelsäure, wobei eine Säurephase ("Absäure") erhalten wird, die durch das Koppelprodukt der Nitrierung, nämlich das Wasser, verdünnt wird. Für die zweite Reaktion zum Dinitrotoluol ist eine höhere Schwefelsäurekonzentration erforderlich als für die erste Reaktion zum Nitrotoluol. Daher gibt man üblicherweise die konzentrierte Schwefelsäure zum zweiten Reaktionsschritt, trennt diese nach erfolgter Reaktion ab und gibt durch die Reaktion verdünnte Schwefelsäure zum ersten Reaktionsschritt zu. Auch hier fällt als Koppelprodukt Wasser an, das die Schwefelsäure weiter verdünnt. Auch bei der ersten Reaktion trennt man die Schwefelsäure ab. Diese wird üblicherweise aufkonzentriert und kann anschließend wieder in der zweiten Reaktionsstufe eingesetzt werden.

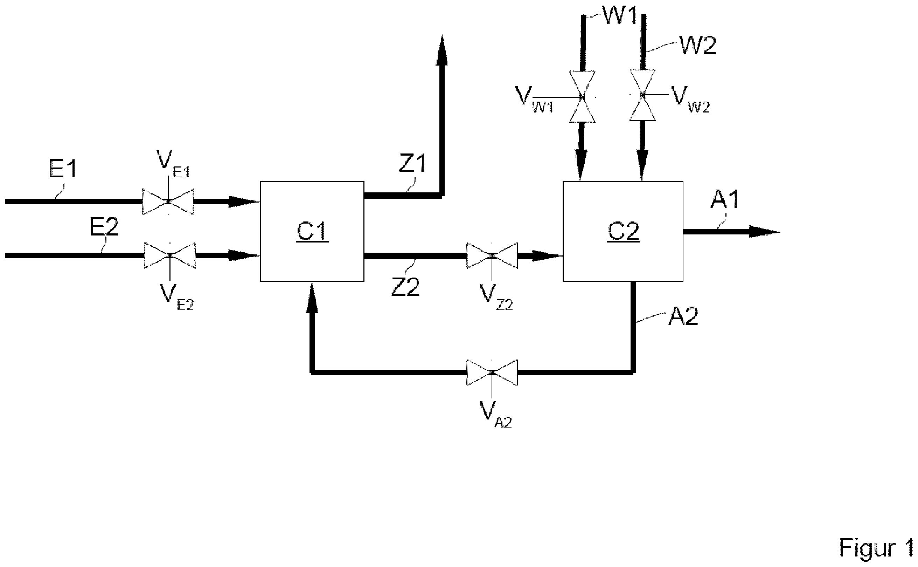

Ein solches Verfahren ist weiter unten in abstrahierter Form anhand der Figur 1 beschrieben, wobei die Stoffe Z1 und A2 als Koppelprodukte zu verstehen sind, da sie aufgrund der Natur der ablaufenden Reaktion in einem vorgegebenen bestimmten Verhältnis zueinander anfallen. Die Kopplung dieser beiden chemischen Reaktionen auf diese Weise hat viele wirtschaftliche und ökologische Vorteile, denn der Anfall an Abfallströmen ist bei dieser Verfahrensweise minimiert. Eine solche Kopplung zweier chemischer Reaktionen bedingt jedoch auch Herausforderungen.

Die Mengenflüsse aller den chemischen Reaktionen zugeführten Stoffen (nachfolgend auch allgemein als Stoffe bezeichnet) müssen genau aufeinander abgestimmt werden. Dies geschieht bislang derart, dass die Soll-Durchflussrate F_{i,soll} oder die Ist-Durchflussrate F_{i,ist} eines ausgewählten Stoffes vorgegeben wird und die Mengenflüsse der übrigen Stoffe relativ zu dieser Durchflussrate auf geeignete Soll-Durchflussraten F_{i,soll} eingeregelt werden. In welchem Verhältnis - natürlich immer in dem von der Stöchiometrie der zugrundeliegenden Reaktionsgleichung gesteckten Rahmen - die Soll-Mengenflüsse F_{i,soll} der übrigen Stoffe idealerweise zur Durchflussrate des vorgegebenen Stoffes stehen sollten, ist für alle gängigen chemischen Reaktionen aus der Patent- und Fachliteratur bekannt und kann ferner durch dem Fachmann bekannte ingenieurtechnische Berechnungen ermittelt werden.

Im Falle einer Produktionsanlage für miteinander gekoppelte Reaktionen erfordert die Inbetriebnahme oder die Einstellung größerer Änderungen der pro Zeiteinheit angestrebten Produktionsmenge daher besondere Umsicht. In der Praxis verfährt man so, dass man den Soll-Mengenfluss eines Eingangsstoffes oder eines Ausgangsstoffes vorgibt (im Verfahren gemäß FIG. 1 z.B. des ersten Eingansstoff E1) und diesen ausgehend vom momentan vorliegenden Ist-Wert durch eine im Wesentlichen lineare Steigerung anfährt, wobei man den Mengenfluss nur langsam steigert. Die Sollwerte der Durchflussraten der übrigen Stoffe werden dann in Relation zu dem Durchflussrate des vorgegebenen Stoffs analog eingestellt.

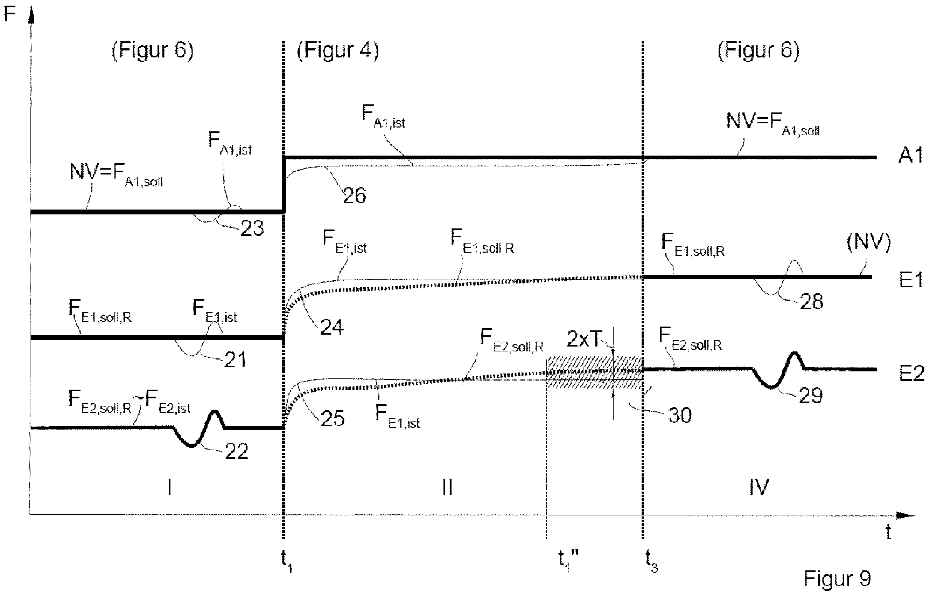

Während eines "Einschwingvorgangs" (was weiter unten anhand der Figur 9 auch erläutert wird) sind mehr oder weniger große Abweichungen der Istwerte von den Sollwerten der Durchflussraten unvermeidbar. Da in Produktionsverfahren mit miteinander gekoppelten Reaktionen eine Vielzahl von Mengenflüssen untereinander abgestimmt werden müssen, ist hier die Gefahr von unerwünschten Abweichungen, die im Extremfall zu einer Unterbrechung des Verfahrens führen können, besonders groß. Dies gilt insbesondere auch deshalb, weil Steigerungen der Mengenflüsse bislang langsam, nämlich in einer Vielzahl von kleiner Steigerungsschritten, vorgenommen werden, wodurch sich die Zahl der möglichen Fehlerquellen multipliziert. Insbesondere kann es dazu kommen, dass die Regler für die verschiedenen Massenströme zeitlich unterschiedliche Regelabweichungen besitzen. Dadurch kann es zu unzulässigen Abweichungen der Stöchiometrie, d.h. der Verhältnisse der Stoffströme zueinander, kommen. Beispielsweise kann ein Regler überschwingen, d.h. der Ist-Wert übersteigt den Sollwert während ein anderer Regler den Ist-Wert nur sehr langsam an den Sollwert heranführt, wodurch der Ist-Wert deutlich kleiner als der Soll-Wert ist. Sofern eine solche Abweichung aus prozessbedingten Gründen unzulässig ist, müsste die Produktionsanlage abgeschaltet werden, wenn die Verhältnisse der Istwerte der Reaktanden außerhalb vorgegebener Intervalle liegen.

Eine mögliche Lösung liegt zwar darin, bei größeren Abweichungen des angestrebten Verhältnisses der Durchflussrate der zugeführten Stoffen zueinander - die aber noch innerhalb bestimmter Grenzwerte liegen - die weitere Steigerung der Durchflussrate des vorgegebenen Stoffes so lange zu unterbrechen, bis sich das System wieder stabilisiert hat. Diese Vorgehensweise hat diverse Nachteile:
- Die langsame Anhebung der Durchflussrate des Ausgangsstoffs bedingt Produktionsverlust.
- Es besteht während solcher kritischer Phasen ein erhöhter Überwachungsaufwand durch das Bedienpersonal.
- Die beschriebene Vorgehensweise ist komplex und weist nur eine geringe Fehlertoleranz auf.

Des Weiteren kann es geboten sein, für einzelne der beteiligten Stoffe größere Volumina an Zwischenspeichern bereitzustellen. Im Beispiel der in FIG. 1 gezeigten Produktionsanlage kann es etwa erforderlich sein, zum Zwecke der Inbetriebnahme eine größere Menge der beiden Koppelprodukte (also des Zwischenstoffes Z2 und des zweiten Ausgangsstoffes A2) in einem Zwischenspeicher vorrätig zu halten. Dies ist, zumindest wenn es sich über einen längeren Zeitraum erstreckt, betriebswirtschaftlich nachteilig und birgt Sicherheitsrisiken. Dies führt zu erhöhten Investitions- und Instandhaltungskosten. Darüber hinaus sind auch durchaus Fälle denkbar, in denen einer solchen Vorgehensweise allein aus technischen Gründen Grenzen gesetzt sind, etwa wenn eines der Koppelprodukte nur begrenzte Zeit stabil ist oder bei längerer Lagerung Materialien des Zwischenspeichers angreift.

Diesen besonderen Herausforderungen im Zusammenhang mit dem Betrieb chemischer Produktionsanlagen für miteinander gekoppelte Reaktionen wird in der einschlägigen Patent- und Fachliteratur bisher allenfalls bedingt Rechnung getragen. Im Zusammenhang mit der Herstellung von Dinitrotoluol durch die miteinander gekoppelten Reaktionen Nitrierung von Toluol zu Mononitroluol (z.B. chemische Reaktion C1 in Figur 1) und Nitrierung von Mononitrotoluol zu Dinitrotoluol (z.B. chemische Reaktion C2 in Figur 1) werden in der Patentliteratur regelmäßig die Verhältnisse der einzelnen Stoffströme diskutiert, ohne jedoch die oben genannte Problematik besonders zu würdigen.
So beschreibt EP 1 880 989 A1 ein Verfahren zur Herstellung von Dinitrotoluol durch Nitrierung von Toloul mit Nitriersäure (Gemisch von Salpetersäure und Schwefelsäure), bei dem in einer ersten Stufe das Toluol zu Mononitrotoluol (MNT), nachfolgend das Mononitrotoluol in einer zweiten Stufe zu Dinitrotoluol (DNT) umgesetzt wird, wobei in der Mononitrierstufe das Gewichts-Verhältnis der wässrigen zur organischen Phase auf Werte > 2 : 1 und in der Dinitrierstufe auf Werte > 1,5 : 1 eingestellt wird und in beiden Schritten jeweils die organische Phase als disperse Phase in der wässrigen Phase (homogene Phase) dispergiert wird und gleichzeitig insgesamt weniger als 2,06 mol Salpetersäure pro mol Toluol in das Verfahren eingespeist werden.

WO 2005/075407 A1 beschreibt ein Verfahren zur Herstellung von Dinitrotoluol, umfassend die Schritte a) Umsetzung von Toluol mit Salpetersäure in Anwesenheit von Schwefelsäure zu Mononitrotoluol, b) Trennung des Reaktionsprodukts aus Schritt a) in eine Mononitrotoluol enthaltende organische Phase und eine Schwefelsäure enthaltende wässrige Phase, c) Umsetzung der Mononitrotoluol enthaltenden organischen Phase mit Salpetersäure in Anwesenheit von Schwefelsäure zu Dinitrotoluol, d) Trennung des Reaktionsprodukts aus Schritt c) in eine Dinitrotoluol enthaltende organische Phase und eine Schwefelsäure enthaltende wässrige Phase, wobei das Reaktionsprodukt aus Schritt a) einen Gehalt an Toluol von 0,1 bis 10 Gew.-%, bezogen auf die organische Phase, und einen Gehalt an Salpetersäure von 0,1 bis 1,2 Gew.-%, bezogen auf die wässrige Phase, aufweist und die Phasentrennung in Schritt b) so erfolgt, dass eine Weiterreaktion des Toluols mit der Salpetersäure verhindert wird.
EP 0 903 336 A2 beschreibt ein Verfahren zur Herstellung von Dinitrotoluol umfassend mindestens zwei Dinitrierungsstufen, wobei als Schwefelsäurebeschickung für die Nitrierungsanlage eine "schwache Säure" verwendet wird. Das Verfahren mit der schwachen Säure verwendet eine Schwefelsäure mit einer Konzentration im Bereich von 86 bis 91 Gew.-%, um den gesamten Schwefelsäurebedarf der Anlage zu decken.
ACS Symposium Series, Vol. 623, Kapitel 21, "Industrial Nitration of Toluene to Dinitrotoluene", offenbart lediglich in ganz allgemeiner Weise, dass die Zuflüsse an Rohmaterialien genau überwacht werden müssen und in bestimmten Fällen aus Sicherheitsgründen Notabschaltungen vorgesehen sind.

Es ist daher Aufgabe der vorliegenden Erfindung, ein verbessertes Verfahren zur kontinuierlichen Herstellung eines Produkts über zumindest zwei miteinander gekoppelte chemische Reaktionen bereitzustellen.

Die der Erfindung zugrunde liegende Aufgabe wird durch ein Verfahren nach Anspruch 1 gelöst; bevorzugte Ausgestaltungen ergeben sich aus den Unteransprüchen und der nachfolgenden Beschreibung.

Erfindungsgemäß ist ein Verfahren zur kontinuierlichen Herstellung eines Produkts über zumindest zwei miteinander gekoppelte chemische Reaktionen vorgesehen. Es werden zumindest zwei Eingangsstoffe einer ersten chemischen Reaktion zugeführt, wobei durch die erste chemische Reaktion aus den Eingangsstoffen eine Mehrzahl von Zwischenstoffen erzeugt wird, und wobei zumindest einer der Zwischenstoffe einer zweiten chemischen Reaktion zugeführt wird. Der der zweiten chemischen Reaktion zumindest eine zugeführte Zwischenstoff wird, insbesondere unter Verwendung zumindest eines weiteren Stoffs, in einer zweiten chemischen Reaktion zu einer Mehrzahl von Ausgangsstoffen weiterverarbeitet, nämlich zum chemischen Produkt und zumindest einem weiteren Ausgangsstoff. Die Durchflussraten der zugeführten Stoffe, die einer der Reaktionen zugeführt werden, werden durch ein jeweiliges Stellglied eingestellt, wobei jedem der zugeführten Stoffe ein separates Stellglied zugeordnet ist, wobei zumindest eines der Stellglieder, insbesondere sämtliche der Stellglieder, mit jeweils einer von einem Regler vorgegebenen Stellgröße beaufschlagt wird. Unter Stellgliedern werden hier Einrichtungen verstanden, die in Abhängigkeit einer Eingangsgröße, z. B. des Ausgangs eines Reglers, eine physikalische Größe, z. B. einen Massestrom, verändern können. Hierzu gehören beispielsweise in Ihrem Öffnungsgrad einstellbare Ventile oder in ihrer Fördermenge einstellbare Pumpen.

Das erfindungsgemäße Verfahren ist dadurch gekennzeichnet, dass für eine Änderung der Produktionsrate des chemischen Produkts zumindest eines dieser Stellglieder während einer Einschwingphase anstelle der von den jeweiligen Reglern vorgegebenen Stellgrößen jeweils mit einer temporären Stellgröße beaufschlagt wird, wobei die temporäre Stellgröße bzw. die temporären Stellgrößen in Abhängigkeit eines Vorgabewertes von zumindest einer Steuerungseinheit erzeugt werden.

Im Rahmen der vorliegenden Erfindung sind zwei chemische Reaktionen miteinander gekoppelt, wenn mindestens ein Koppelprodukt einer chemischen Reaktion, ggf. nach Aufarbeitung, als Reaktionspartner in der einer anderen chemischen Reaktion eingesetzt wird. Dabei wird unter einem Koppelprodukt einer chemischen Reaktion ein solches Produkt verstanden, das neben dem angestrebten Zielprodukt der Reaktion infolge der naturgesetzlich vorgegebenen Stöchiometrie der zugrundeliegenden Reaktionsgleichung zwangsläufig mitgebildet wird. Im Rahmen der Herstellung von Dinitrotoluol handelt es sich bei diesem Koppelprodukt um das während der Nitrierreaktion gebildete Wasser, das von der Schwefelsäure aufgenommen wird. Dies ist zu unterscheiden von Nebenprodukten, deren Bildung durch verbesserte Reaktionsbedingungen, geeignete Wahl des Katalysators und dergleichen Maßnahmen mehr zumindest minimiert werden kann.

Das hier beanspruchte Verfahren betrifft folglich die Durchführung einer Änderung der Produktionsrate. Unter der Produktionsrate wird insbesondere eine gewünschte Durchflussrate, also der Sollwert der Durchflussrate eines am Prozess beteiligten Stoffs verstanden. Insbesondere ist dies auch anwendbar bei einer Änderung, insbesondere Erhöhung, der Produktionsrate von zumindest 30%, vorzugsweise zumindest 50% und insbesondere beim Hochfahren einer Produktionsanlage aus dem Ruhezustand.

In einem Ausgangszustand weist die Produktionsrate einen anderen Wert auf als in einem Endzustand. Insbesondere kann das Verfahren angewendet werden beim Hochfahren des Herstellungsprozesses; dann beträgt die Produktionsrate im Ausgangszustand 0 und im Endzustand ist die Produktionsrate, insbesondere die Nenn-Kapazität einer Produktionsanlage, von 0 verschieden. Alternativ kann die Produktionsanlage etwa aus Nachfragegründen für einen bestimmten Zeitraum nur mit verringerter, beispielsweise mit der halben Nenn-Kapazität betrieben werden, und soll dann möglichst rasch wieder auf Nenn-Kapazität hochgefahren werden. Der umgekehrte Fall einer signifikanten Verringerung der aktuellen Produktionskapazität ist ebenfalls von dem erfindungsgemäßen Verfahren mit umfasst.

Der Kern der Erfindung liegt nun insbesondere darin, dass zur Änderung der Produktionsrate nun die Regler (d. h. zumindest einer) vorübergehend außer Kraft gesetzt werden und durch eine oder mehrere Steuerungen ersetzt werden. Die Steuerung erzeugt nun die Stellgrößen basierend auf einem Vorgabewert. Der Vorgabewert steht insbesondere in Relation zur Änderung der Produktionsrate. Bei dem Vorgabewert kann es sich insbesondere um den Sollwert der Durchflussrate eines Stoffes handeln, der einer der chemischen Reaktionen zugeführt wird; Bei dem Vorgabewert kann es sich alternativ um den Sollwert der Durchflussrate eines Ausgangsstoffes handelt, der durch eine chemischen Reaktionen erzeugt wird.

Wenn genau ein Stellglied während der Einschwingphase mit einer solchen temporären Stellgröße beaufschlagt wird, handelt es sich bevorzugt um ein Stellglied, mit dem die Durchflussrate eines Stoffes (insbesondere ein Eingangsstoff) eingestellt wird, der einer der chemischen Reaktionen zugeführt wird. Vorzugsweise stellt die Nutzervorgabe dann den Sollwert des Durchflusses dieses Stoffes dar. Im Falle der Dinitortoluolherstellung stellt vorzugweise der neue Sollwert der Durchflussrate von Toluol die Nutzervorgabe dar.

Durch das erfindungsgemäße Vorgehen kann eine schlagartige Veränderung, insbesondere Erhöhung, der Istwerte der Durchflussraten der einzelnen Stoffe erzielt werden, die bereits nach wenigen Sekunden Werte annehmen, die recht nahe an den Durchflussraten des zu erreichenden stationären Zustands liegen. Durch die Überbrückung der vom Regler ausgegebenen Stellgrößen durch die temporären Stellgrößen kann jeder Regler für sich genommen einschwingen, ohne dass das die Regler irgendeinen Einfluss auf den Prozess haben. Dabei kommt es diesem Einschwingvorgang zugute, dass durch die Vorgabe der temporären Stellgrößen die Istwerte bereits recht schnell einen stationären Zustand einnehmen; da die Sollwerte einiger Regelkreise in einem solch verschachtelten Herstellungsverfahren in der Regel von (nunmehr stationären) Istwerten anderer Regelkreise abhängen, wird das Einschwingen begünstigt.

In einer bevorzugten Ausgestaltung können auch sämtliche Stellglieder auf diese Weise mit Stellgrößen beaufschlagt werden. Dies kann den Einschwingvorgang nochmals beschleunigen.

Vorzugsweise werden die Stellgrößen durch zumindest eine Steuerungsfunktion der Steuerungseinheit mithilfe einer Datenbasis erzeugt, in der mathematische Zuordnungen zwischen einer temporären Durchflussrate und einer dazugehörenden Stellgröße für jedes dieser Stellglieder hinterlegt sind. Die Steuerungseinheit bedient sich so während der Einschwingphase einem zuvor hinterlegten Wissen, beispielsweise der Art
*"Wenn das Ventil derart weit aufgedreht ist (Stellgröße), dann fließt eine bestimmte Menge Stoff (Durchflussrate) durch dieses Ventil".*
Diese Zuordnung ermöglicht es der Steuerungseinheit, das Ventil ausreichend gut auf einen gewünschten Sollwert einzustellen, wobei es in dieser Phase eben nicht darauf ankommt, dass der Istwert der Durchflussrate dem Sollwert exakt entspricht. Vielmehr ist in dieser Einschwingphase bedeutsam, den Prozess auf hohem Niveau zunächst in einen stationären Zustand zu bringen. Durch das im Wesentlichen gleichzeitige Verstellen sämtlicher relevanter Stellglieder entsprechend der zu erwartenden Durchflussraten, kann binnen kürzester Zeit, insbesondere innerhalb weniger Sekunden, ein solcher stationärer Zustand erreicht werden.

Vorzugsweise wird für jeden der zugeführten Stoffe der Sollwert einer temporären Durchflussrate unter Berücksichtigung des Vorgabewertes, insbesondere anhand einer stöchiometrischen Funktion, erzeugt, wobei dieser Sollwert der Erzeugung der zugehörigen temporären Stellgröße zugrunde gelegt wird. Wie im stationären Regelbetrieb müssen auch während der Einschwingphase die Durchflussraten der zugeführten Stoffe zumindest einigermaßen aufeinander abgestimmt sein, damit die Regler Gelegenheit zum Einschwingen bekommen. Die stöchiometrische Funktion erzeugt dabei zueinander passende Sollwerte der Durchflussraten der einzelnen Stoffe, entsprechendem dem Mengenverhältnis, welches die chemischen Reaktionen erfordern. Die stöchiometrische Funktion kann dabei auf weitere Messwerte des Prozesses zugreifen, insbesondere auf Werte, die auf die Konzentration der Stoffe Rückschlüsse zulassen, beispielsweise die Dichte.

Vorzugsweise wird nach Ablauf einer Einschwingphase umgeschaltet von der Beaufschlagung dieser Stellglieder mit den temporären Stellgrößen auf eine Beaufschlagung mit den von den jeweiligen Reglern vorgegebenen Stellgrößen. Sind die Regler nun eingeschwungen, so übernehmen sie wieder die Stellgrößenvorgabe. Da die Differenz zwischen den Istwerten und den Sollwerten (der Regler) der Durchflussraten nunmehr recht gering ist, kann die nun noch vorliegende Regeldifferenz problemlos durch die einzelnen Regelkreise korrigiert werden.

Der Ablauf der Einschwingphase, so dass die Stellgrößenvorgabe umgestellt werden kann, ist vorzugsweise dann erreicht ist, wenn sämtliche Regler eine Stellgröße ausgeben, deren Schwankungsbreiten unterhalb vorgegebener Schwellwerte liegen.

Das Verfahren eignet sich insbesondere für die Herstellung von Dinitrotoluol, was anhand des Ausführungsbeispiels noch näher erläutert wird.

Die Erfindung betrifft ferner die Verwendung einer chemischen Produktionsanlage zur Durchführung des erfindungsgemäßen Verfahrens.

Die Erfindung betrifft ferner eine Regelungs- und Steuerungsanordnung, die ausgebildet ist, das Verfahren der vorgenannten Art zu regeln und steuern. Die Anordnung umfasst eine Steuerungseinheit sowie mehrere Regler zur wechselnden Beaufschlagung von Stellgliedern, zur Beeinflussung der Durchflussrate eines fließenden Stoffs, mit einer Stellgröße, eine Datenbasis zur Hinterlegung von Zuordnungen zwischen Stellgrößen und Durchflussraten, anhand derer die Steuerungseinheit temporäre Stellgrößen in Abhängigkeit von Sollwerten der Durchflussraten ausgibt.

Die Erfindung wird anhand der Figuren nachfolgend erläutert, hierin zeigt:
- **Figur 1**: ein Prozessschaubild eines herkömmlichen Verfahrens zur kontinuierlichen Herstellung eines chemischen Produkts;
- **Figur 2**: zwei Prozessschaubilder zur Erläuterung der Regelung der Stoffströme im Verfahren entsprechend Figur 1;
- **Figur 3**: das Prozessschaubild nach Figur 2a, ergänzt um Recheneinheit zur Ermittlung von Sollwerten;
- **Figur 4**: das Prozessschaubild nach Figur 3, ergänzt um Steuerungseinheit zur Erzeugung von temporären Stellgrößen und Sollwerten;
- **Figur 5**: das Prozessschaubild nach Figur 4, in einem nachfolgenden Verfahrensschritt;
- **Figur 6**: das Prozessschaubild nach Figur 5, in einem nachfolgenden Verfahrensschritt;
- **Figur 7**: Die Steuerungseinheit der Figuren 4 bis 6 in Einzelheit;
- **Figur 8**: ein Diagramm mit Durchflusswerten während der Durchführung des erfindungsgemäßen Verfahrens;
- **Figur 9**: ein Diagramm mit Durchflusswerten während der Durchführung eines alternativen erfindungsgemäßen Verfahrens;
- **Figur 10**: ein Diagramm mit Durchflusswerten während der Durchführung eines Verfahrens nach dem Stand der Technik.

Figur 1 zeigt schematisch ein Prozessschaubild eines Verfahrens zur kontinuierlichen Herstellung eines chemischen Produkts. Das herzustellende chemische Produkt ist in diesem Beispiel ein erster Ausgangsstoff A1.

Es werden zumindest zwei Eingangsstoffe E1, E2 verwendet, die einer ersten chemischen Reaktion C1 zugeführt werden. Über Stellglieder V_{E1,} V_{E2}, beispielsweise Durchflussventile, wird die Durchflussrate (im weiteren Verlauf der Beschreibung mit dem Bezugszeichen F versehen) der jeweils zugeführten Eingangsstoffe E1, E2 eingestellt. In der ersten chemischen Reaktion C1 werden die Eingangsstoffe E1, E2 zu mehreren Zwischenstoffen verarbeitet, im vorliegenden Fall zwei Zwischenstoffe Z1, Z2.

Einer der Zwischenstoffe, hier der zweite Zwischenstoff Z2, wird direkt einer zweiten chemischen Reaktion C2 zugeführt. Die Durchflussflussrate des zweiten Zwischenstoffs Z2 wird über ein Stellglied V_{Z2} eingestellt. Ferner werden der zweiten chemischen Reaktion C2 noch weitere Stoffe W1, W2 zugeführt. Auch die Durchflussraten der weiteren Stoffe werden über Stellglieder V_{W1}, V_{W2} eingestellt. Einer der weiteren Stoffe W1, W2 kann wiederum durch Aufarbeitung aus dem ersten Zwischenstoff Z1 hergestellt werden.

Die zweite chemische Reaktion C2 verarbeitet nun die ihr zugeführten Stoffe W1, W2 in die beiden Ausgangsstoffe A1, A2. Während der erste Ausgangsstoff A1 das letztlich herzustellende chemische Produkt darstellt, wird der zweite Ausgangsstoff A2 wieder der ersten chemischen Reaktion C1 zugeführt. Dies kann unmittelbar geschehen, wie in Figur 1 dargestellt; alternativ kann der zweite Ausgangsstoff A2 auch mit einem der anderen Eingangsstoffe vor der Zuführung in die erste chemische Reaktion vermischt werden (nicht dargestellt).

Die Besonderheit und gleichzeitig die Schwierigkeit bei der Durchführung solcher Verfahren liegt darin, dass den chemischen Reaktionen C1 und/oder C2 jeweils zumindest ein Stoff (A2, Z2) zugeführt wird, welcher erst in einer der vorhergehenden chemischen Reaktionen C2 bzw. C1 erzeugt wird. Wenn also die Produktionsrate einer chemischen Reaktion, beispielsweise der zweiten chemischen Reaktion C2 zur letztendlichen Herstellung des ersten Ausgangsstoffs A1 erhöht, beispielsweise verdoppelt, werden soll, so ist es zwingend erforderlich, dass auch der ihr zugeführte Stoff Z2 (sowie die anderen Stoffe), verbunden mit einer entsprechend erhöhten Produktionsrate der ersten chemischen Reaktion C1, bereitgestellt wird.

Im vorliegenden Fall benötigt zudem die erste chemische Reaktion C1 den zweiten Ausgangsstoff A2 ebenfalls als einen zuzuführenden Stoff, wobei der zweite Ausgangsstoff A2 erst durch die zweite chemische Reaktion C2 hergestellt wird. Es ergibt sich folglich eine Rückkopplung, so dass jede der chemischen Reaktionen C1, C2 abhängig von den Produkten der jeweils anderen chemischen Reaktion C2, C1 ist.

Aufgrund von z.B. Verschleiß in den Stellgliedern und Änderungen in den Führungsgrößen unterliegen die tatsächlichen Durchflussraten der Stoffe stets Schwankungen, die innerhalb vorgegebener Grenzwerte (einschließlich Grenzwertintervallen) tolerierbar sind. Solche Grenzwerte können durch automatische oder manuelle Eingabe oder Änderung von Parametern vorgegeben werden, insbesondere anhand weiterer Regelbausteine, z.B. Standregler. Grenzwerte können vorgesehen sein für Abweichungen von zueinander redundanten Flussmessungen desselben Stoffstromes oder Abweichungen zwischen Soll- und Istwerten der Durchflussraten einzelner Stoffströme. Die zulässigen Grenzwerte können für jeden Stoff unterschiedlich festgelegt sein. Ein erhöhter Wert an Schwefelsäure ist beispielsweise bei der Nitrierung von Toluol weniger schädlich als ein erhöhter Wert von Salpetersäure. Eine Überschreitung solcher Grenzen kann im Extremfall zur Verriegelung der gesamten Produktionsanlage führen.

Ein solches Verfahren wird insbesondere verwendet zur Herstellung von Dinitrotoluol als erstem Ausgangsstoff A1. Der erste Eingangsstoff E1 ist Toluol, der zweite Eingangsstoff E2 ist Salpetersäure, der erste Zwischenstoff Z1 ist eine Absäure (d. h. im Wesentlichen verdünnte Schwefelsäure), der zweite Zwischenstoff Z2 ist Mononitrotoluol, der zweite Ausgangsstoff A2 ist verdünnte Schwefelsäure, der erste weitere Stoff W1 ist Schwefelsäure und der zweite weitere Stoff W2 ist Salpetersäure. In der ersten chemischen Reaktion C1 erfolgt eine Nitrierung von Toluol E1, Salpetersäure E2 und Schwefelsäure A2 und einer anschließenden Phasentrennung in im Wesentlichen Mononitrotoluol und im Wesentlichen Absäure. In der zweiten chemischen Reaktion erfolgt eine Nitrierung des im Wesentlichen Mononitrotoluols Z2 mit Salpetersäure W2 und Schwefelsäure W1 und einer anschließenden Phasentrennung zu Dinitrotoluol A1 und im Wesentlichen Schwefelsäure A2. Die aus der ersten chemischen Reaktion stammende Absäure Z1 kann aufgearbeitet, insbesondere aufkonzentriert werden, zu der Schwefelsäure W1, die wiederum der zweiten chemischen Reaktion zugeführt wird.

In herkömmlichen Verfahren wurde dies beispielsweise dadurch gelöst, dass die unterschiedlichen Stoffe bzw. Produkte, die einem Prozess zugeführt werden sollen, stets in ausreichenden Mengen in Zwischenspeichern vorgehalten werden. Etwaige Differenzen zwischen dem Bedarf einer chemischen Reaktion zur derzeit aktuellen Produktionsrate der vorhergehenden chemischen Reaktion kann durch einen solchen Zwischenspeicher ausgeglichen werden. Dies ist allerdings eine eher unerwünschte Lösung dieser Problematik, da große Mengen an zwischengespeicherten Stoffen auch Sicherheitsrisiken darstellen und/oder hohe Kosten verursachen. Grundsätzlich besteht daher Bedarf, die Menge an zwischengespeicherten Stoffen möglichst gering zu halten.

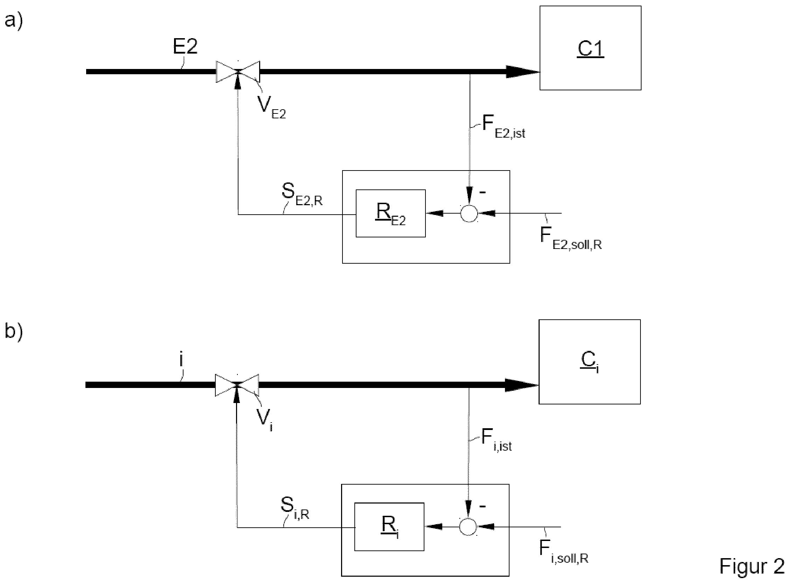

Anhand Figur 2 wird nun erläutert, wie die Regelung der Durchflussrate eines Stoffs erfolgt. Der Begriff Durchflussrate steht dabei stellvertretend für die durchflossene Menge eines Stoffes, unabhängig davon, ob sie in Masse pro Zeiteinheit oder Volumen pro Zeiteinheit usw. erfasst wird.

In Figur 2a wird die Durchflussregelung beispielhaft anhand des zweiten Eingangsstoffs E2 erläutert. Der zweite Eingangsstoff 2 soll mit einer bestimmten Durchflussrate F_{E2,soll,R} der ersten chemischen Reaktion C1 zugeführt werden. Ein nicht dargestelltes Durchflussmessgerät stellt den Istwert F_{E2,ist} einer momentanen Durchflussrate zur Verfügung. Eine Regeldifferenz wird erzeugt durch Vergleich dieses Istwerts F_{E2,ist} mit dem Sollwert F_{E2,soll,R}. Ein Regler R_{E2} gibt basierend auf dieser Regeldifferenz nun eine Stellgröße S_{E,2R} an das Stellglied V_{E2} aus und beaufschlagt so dieses Stellglied mit dieser Stellgröße. Das Stellglied V_{E2} erhöht oder verringert nun entsprechend die tatsächliche Durchflussrate F_{E2,ist}. Als Durchflussmessgeräte können u.a. Ultraschalldurchflussmessgeräte, Massendurchflussmessgeräte, induktive Durchflussmessgeräte oder Staudruckmessgeräte verwendet werden. Ein solches Messgerät kann auch abweichend zur Darstellung nach Figur 2 in Strömungsrichtung betrachtet vor dem Stellglied V_{E2} angeordnet sein. Aufgrund des in der Regel höheren Drucks vor dem Stellglied V_{E2} können die Istwerte dort zuverlässiger gemessen werden, da insbesondere Messwertverfälschungen aufgrund von Gasphasenbildungen vermieden werden. Die Durchflussmessungen werden bevorzugt redundant für jeweils einen Durchflusswert durchgeführt, insbesondere durch Durchführung von mehreren, zeitlich parallelen Messungen.

Der für solche Anwendungen bevorzugte Regler R_{E2} umfasst einen Integralanteil. Es handelt sich vorzugsweise um einem PID-Regler, da solche Regler nicht nur die momentane Durchflussrate möglichst gut auf den Sollwert einregeln, sondern langfristig auch die Gesamtmenge an durchflossener Stoffmenge exakt einregeln können.

Figur 2b ist im Wesentlichen eine Kopie des in Figur 2a dargestellten Regelkreises. Abweichend zur Darstellung nach Figur 2 sind allerdings allgemeine Bezugszeichen verwendet worden, um zu verdeutlichen, dass der in Figur 2a dargestellte Regelkreis auch für die Durchflussregelung sämtlicher anderen zugeführten Stoffe, also des anderen Eingangsstoffs E1, der weiteren Stoffe W1, W2, des zweiten Zwischenstoffs Z2, sowie des zweiten Ausgangsstoffes A2, anwendbar ist, die jeweils in geregelten Mengen den beiden chemischen Reaktionen zuzuführen sind. Grundsätzlich steht der Index i also stellvertretend für die Stoffe E1, E2, W1, W2, Z2, A2, deren jeweilige Durchflussraten Fᵢ zu regeln sind.

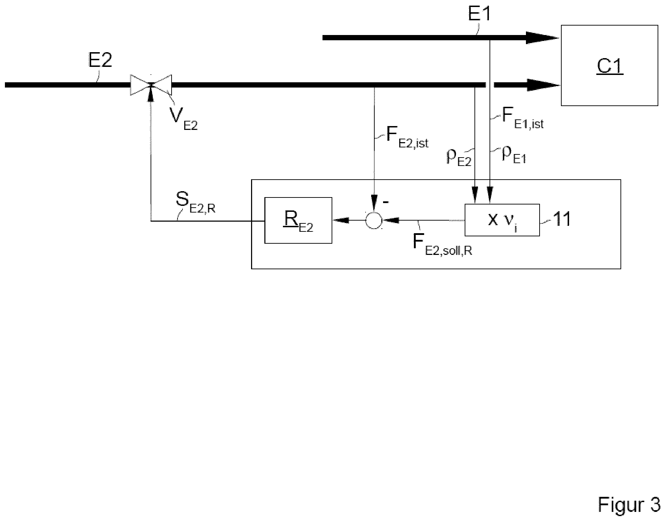

Im stationären Regelbetrieb ist die Produktionsrate des Ausgangsstoffs im Wesentlichen konstant. Im Wesentlichen werden nun die chemischen Reaktionen mit gleichbleibenden Produktionsraten betrieben. Das bedeutet, dass die chemischen Reaktionen im Wesentlichen mit einem konstanten Zufluss an entsprechenden Stoffen versorgt werden müssen. Hierfür ist es erforderlich, dass die Durchflussraten sämtlicher Stoffe geregelt werden. In Figur 3 ist nun beispielhaft für den Stoff E2 gezeigt, wie dessen Durchflussrate F_{E2,ist} in Abhängigkeit der Durchflussrate eines anderen Stoffs (beispielhaft die momentane Durchflussrate F_{E1,ist} des anderen Eingangsstoffs E1) geregelt wird.

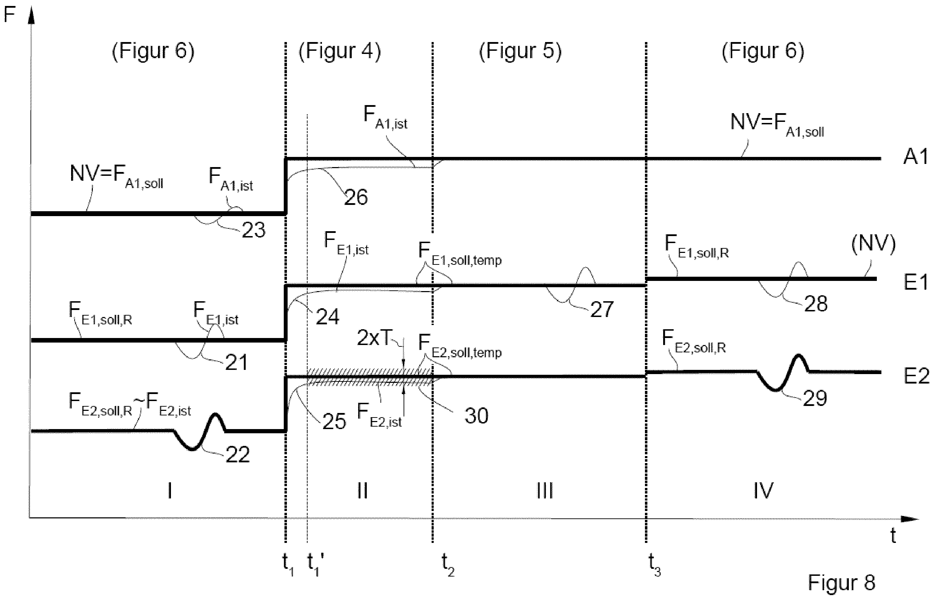

Es wird von dem nicht dargestellten Durchflussmessgerät der Istwert F_{E1,ist} der Durchflussrate des anderen Eingangsstoffs E1 gemessen und einer Recheneinheit 11 zugeführt. Diese Recheneinheit 11 berechnet nun die notwendige Durchflussrate für den zweiten Eingangsstoff E2, was dem Sollwert F_{E2,soll,R} der Durchflussrate entspricht, analog Figur 2a. Diese Berechnung erfolgt unter Berücksichtigung der stöchiometrischen Bedingungen innerhalb der ersten chemischen Reaktion C1. In Figur 3 ist dies vereinfacht dargestellt durch eine Multiplikation des stöchiometrischen Koeffizienten νᵢ mit dem Istwert F_{E1,ist} der Durchflussrate des anderen Eingangsstoffs E1. Sollten sich Schwankungen in der Durchflussrate F_{E1,ist} des ersten Eingangsstoffs E1 ergeben, so werden diese Abweichungen in der Durchflussrate durch den Regelkreis unter Berücksichtigung des stöchiometrischen Koeffizienten kurzfristig auch auf den Sollwert der Durchflussrate F_{E2,soll} des zweiten Eingangsstoffs E2 abgebildet (siehe weiter unten Bezugszeichen 21,22 und 28,29 in Figur 8). Die tatsächliche Durchflussrate F_{E2,ist} des zweiten Eingangsstoffs E2 folgt dann der tatsächlichen Durchflussrate F_{E1,ist} des ersten Eingangsstoffs E1. Die Durchflussrate des ersten Eingangsstoffs E1 wiederum kann in Abhängigkeit der Durchflussrate eines anderen im Herstellungsverfahren beteiligten Stoffes, insbesondere anhand eines gewünschten Ausstoßes F_{A1,soll} an erstem Ausgangsstoffs A1, geregelt werden.

Die Berechnung der Sollwerte durch die Recheneinheit 11 kann ferner unter Einbezug weiterer chemischer und/oder physikalischer Größen erfolgen. Dargestellt ist, dass zu den Stoffströmen der Stoffe E1 und E2 die jeweilige Dichte ρ_{E1} und ρ_{E2} ermittelt wird und an die Recheneinheit 11 übertragen werden. Anhand der Dichte kann die Konzentration der jeweiligen Stoffe berechnet werden, was bedeutsam ist für die erforderliche Durchflussrate. Bei der Herstellung von Dinitrotoluol, was weiter unten noch näher beschrieben wird, können anhand der Dichte bereits Anteile von Dinitrotoluol im zweiten Zwischenstoff festgestellt werden. Entsprechend kann die Zugabe von Salpetersäure (zweiter weiterer Stoff W2) reduziert werden, je größer der Anteil von Dinitrotoluol im zweiten Zwischenstoff Z2 ist. Diese Ausführungen implizieren, dass die hier als Stoffe bezeichneten Produkte, keine sortenreinen Stoffe sein müssen.

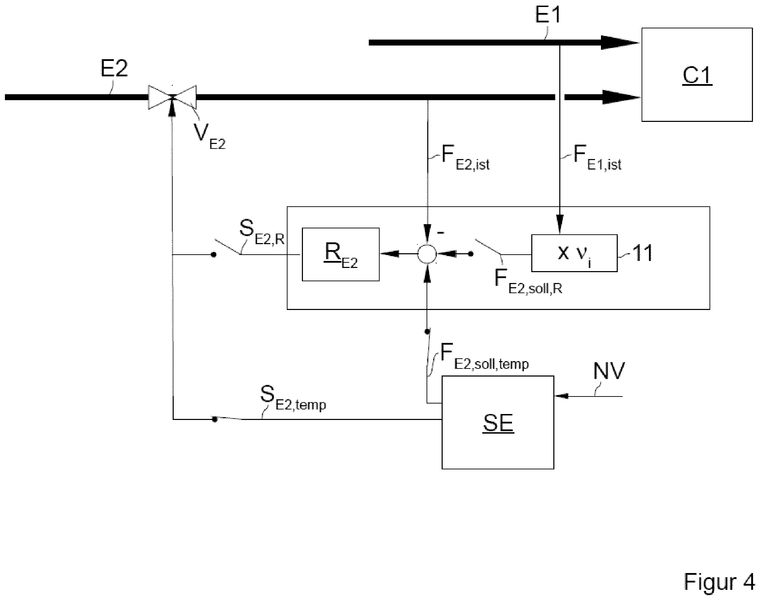

Anhand der Figuren 4 bis 7 wird nun erläutert, welche Maßnahmen zur schlagartigen Erhöhung der Produktionsrate durchgeführt werden. Dieses Verfahren wird insbesondere dann angewendet, wenn nach einem Stillstand der Anlage die Produktion des Ausgangsstoffs wieder aufgenommen werden soll. Auf die Darstellung der Dichte ρ der Stoffe E1 und E2 wird in den weitere Figuren aus Gründen der Übersichtlichkeit verzichtet.

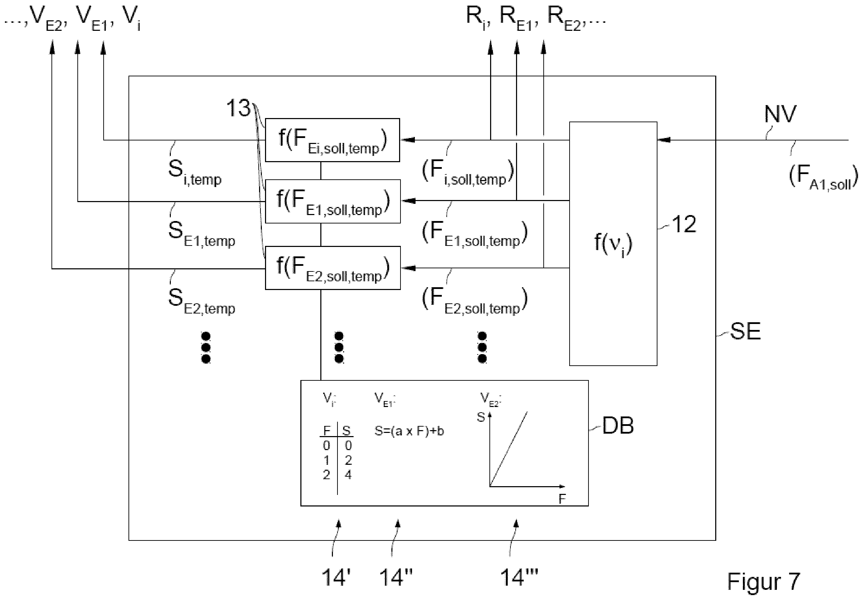

Figur 4 basiert auf dem Regelkreis, der in Figur 3 gezeigt ist. Allerdings ist zu erkennen, dass die Datenverbindung zwischen dem Regler R_{E2} und dem Stellglied V_{E2} unterbrochen ist. Das bedeutet, dass das Stellglied V_{E2} nicht mit der vom Regelkreis vorgegebenen Stellgröße S_{E2,R} des Reglers R_{E2} beaufschlagt wird. Stattdessen wird das Stellglied V_{E2} mit einer temporären Stellgröße S_{E2,temp} beaufschlagt, die von einer Steuerungseinheit SE ausgegeben wird. Es wird folglich die vom Regler ausgegebene Stellgröße _{SE2,R} durch die von der Steuerungseinheit SE ausgegebene temporäre Stellgröße S_{E2,temp} überschrieben. Die Berechnung der temporären Stellgröße S_{E2,temp} basiert wiederum auf einem Eingangswert der Steuerungseinheit SE, beispielsweise einer Nutzervorgabe NV, welche z.B. ein gewünschter Sollwert für die Produktionsrate des ersten Ausgangstoffs A1 ist. Die Berechnung hierzu wird weiter unten anhand der Figur 7 erläutert.

Gleichzeitig wird auch von der Steuerungseinheit SE ein temporärer Sollwert F_{E2,soll,temp} der Durchflussrate des zweiten Eingangsstoffs E2 ausgegeben, die Eingang findet in den Regler R_{E2}. Der Regler R_{E2} wird folglich in einer Art Leerlaufmodus betrieben, in dem zwar eine Stellgröße S_{E2,R} vom Regler R_{E2} ausgegeben bzw. erzeugt wird; diese Stellgröße S_{E2,R} wird aber (noch) nicht an das Stellglied V_{E2} ausgegeben. Das Besondere ist auch hier, dass der Regler R_{E2} nicht auf Basis des Sollwerts F_{E2,soll,R} arbeitet, der von dem Prozess vorgegeben wird, sondern auf Basis des Sollwerts F_{E2,soll,temp} arbeitet, der von der Steuereinheit SE vorgegeben wird, was ebenfalls weiter unten noch näher erläutert wird.

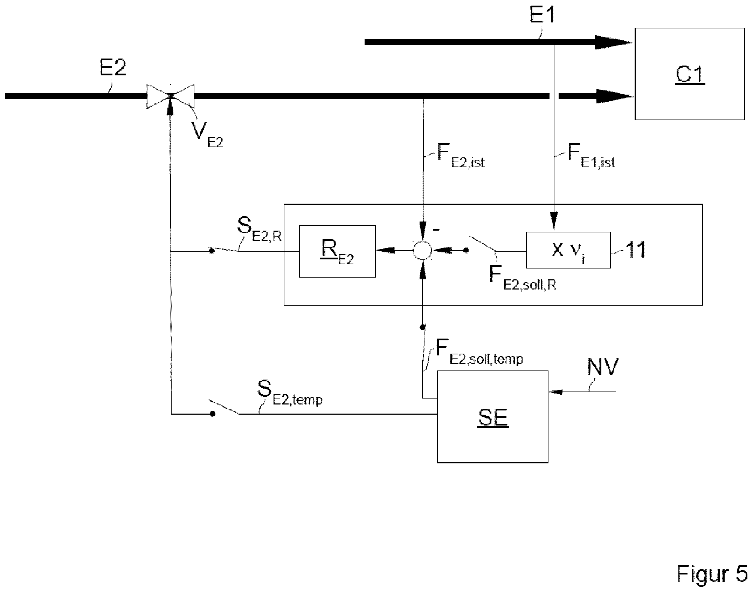

In einem zweiten Schritt wird nun, wie in Figur 5 dargestellt, das Stellglied V_{E2} nun nicht mehr mit der temporären Stellgröße S_{E2,temp} beaufschlagt, sondern mit der Stellgröße S_{E2,R}, die vom Regler R_{E2} ausgegeben wird. Die Besonderheit bleibt allerdings bestehen, dass der Regler R_{E2} nach wie vor durch mit dem Sollwert F_{E2,soll,temp} von der Steuerungseinheit SE versorgt wird, ohne dass die Durchflussraten anderer Stoffe berücksichtigt werden.

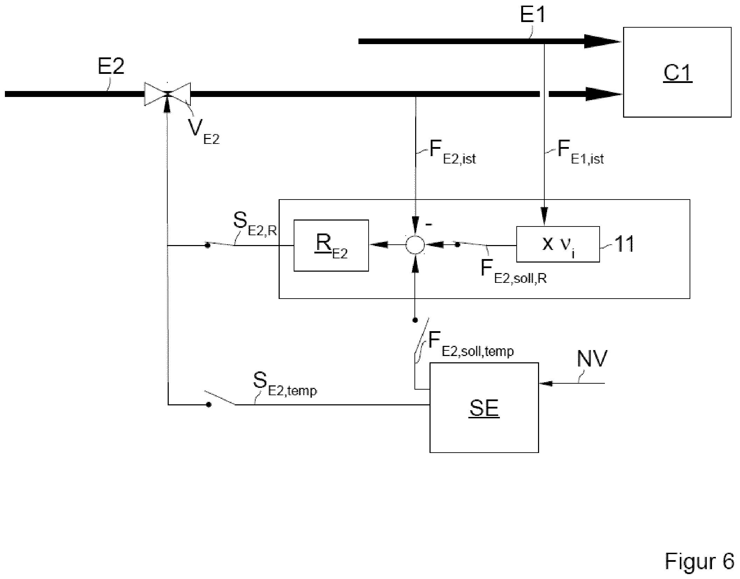

In einem dritten Schritt, wie in Figur 6 dargestellt, wird nun auch der Sollwert, der in den Regler R_{E2} Eingang findet, umgestellt auf den vom Prozess vorgegebenen Sollwert F_{E2,soll,R}; sämtliche von der Steuerungseinheit SE vorgegebenen Werte, nämlich der Sollwert F_{E2,soll,temp} sowie die Stellgröße S_{E2,temp} bleiben ab jetzt wieder unberücksichtigt. Im Wesentlichen entspricht dies nun dem Schaltplan nach Figur 3, da der Prozess nun vollständig von der Steuerungseinheit SE entkoppelt ist.

Anhand der Figur 7 wird nun die Funktionsweise der Steuerungseinheit SE, wie sie in den Figuren 4 bis 6 gezeigt ist, näher erläutert. Auf der rechten Seite ist die Nutzervorgabe NV als Eingangswert erkennbar. Die Nutzervorgabe kann beispielsweise eine gewünschte Produktionsrate F_{A1,soll} des herzustellenden Produkts A1 sein. Wenn nun bekannt ist, welche Menge dieses Produkts hergestellt werden soll, so lassen sich aufgrund der stöchiometrischen Bedingungen (und insbesondere unter Einbezug weiterer physikalischer oder chemischer Größen der Stoffe) die erforderlichen Sollwerte für sämtliche im Prozess benötigten Stoffe rechnerisch ermitteln, was durch den Funktionsblock 12 durchgeführt wird. Ausgangswerte dieses Funktionsblocks 12 sind nun die temporären Sollwerte F_{i,soll,temp}, für jeden der wesentlichen Stoffe, deren Durchflussraten im Prozess geregelt werden. Beispielhaft und stellvertretend für andere Stoffe sind die Sollwerte F_{E1,soll,temp}, F_{E2,soll,temp} der Durchflussraten der beiden Eingangstoffe dargestellt. Analog gilt diese Darstellung auch für den Zwischenstoff Z2 sowie die weiteren Stoffe W1, W2. Der Funktionsblock 12 verwendet analog zur Recheneinheit 11 (Figur 3) auch weitere chemische und/oder physikalische Größen zur Ermittlung der Sollwerte, wie beispielsweise Werte insbesondere die Dichte p, die Rückschlüsse auf die Konzentration der einzelnen Stoffe zulassen.

Diese temporär vorgegebenen Sollwerte F_{i,soll,temp}, F_{E1,soll,temp}, F_{E2,soll,temp} werden nun jeweils einer Steuerungsfunktion 13 zugeleitet. Die Steuerungsfunktion 13 berechnet auf Basis des vorgegebenen temporären Sollwerts F_{i,soll,temp}, F_{E1,soll,temp}, F_{E2,soll,temp} der Durchflussrate jeweils eine temporäre Stellgröße S_{i,temp}, S_{E1,temp}, S_{E2,temp} für sämtliche relevanten Stellglieder Vᵢ, durch die sich die Durchflussraten der wesentlichen Stoffe einstellen lassen. Die temporären Stellgrößen werden allerdings nicht eingeregelt, wie sonst üblich; vielmehr werden die Stellgrößen mithilfe einer Datenbasis DB erzeugt, in denen eine Vielzahl von Stellgrößen oder Berechnungsparametern zur Berechnung solcher Stellgrößen abgelegt sind.

Beispielhaft ist eine solche Datenbasis DB in der Figur 7 skizziert. So ist beispielhaft für das Stellglied Vᵢ eine Zuordnungstabelle 14' mit Durchflusswerten F und den dazugehörigen Stellgrößen S dargestellt. Soll beispielsweise der Durchflusswert 0 sein, so ist die Stellgröße 0 auszugeben; bei einer einzustellenden Durchflussrate von 1 ist die Stellgröße 2 auszugeben, bei einer einzustellenden Durchflussrate von 2 ist die Stellgröße 4 auszugeben. Entsprechendes ist auch für alle weiteren relevanten Stellglieder vorhanden. Zwischenwerte können durch Interpolation errechnet werden. Eine solche mathematische Zuordnung muss nicht tabellarisch vorliegen; es können auch Berechnungsformeln 14" oder Kennfelder 14'" hinterlegt sein, die dann rechnerisch ausgewertet werden. Diese Zuordnungen 14 können durch experimentelle Ermittlung oder eine ingenieurtechnische Berechnung erzeugt werden.

Solche Zuordnungen 14 müssen nicht zwangsläufig statisch hinterlegt sein, sondern können dynamisch aktualisiert werden. Dafür können aktuelle Istwerte mit aktuellen Stellgrößen verglichen werden; basierend hierauf kann eine Aktualisierung der in der Datenbasis DB hinterlegten Zuordnungen vorgenommen werden.

Die Zuordnungen können zudem auch weitere Abhängigkeiten umfassen. So wird im Prozess eine Pumpe zum Fördern eines Stoffs verwendet, der auch in anderen Herstellungsverfahren benötigt wird. Je nachdem, wie hoch die Auslastung der Pumpe ist, können sich Druckunterschiede in der Zuführleitung eines Stoffes ergeben, was eine Abhängigkeit der Stellgröße neben der Soll-Durchflussrate auch von dem Druck in der Leitung erforderlich macht.

Analog zur schlagartigen Änderungen der Produktionsrate und der damit verbundenen Änderung der Durchflussraten kann auch die Regelung des Kühlmittelstroms von der Temperaturregelung auf eine Steuerung umgestellt werden. Die erforderlichen Zuordnungen können ebenfalls in der Datenbasis DB abgelegt sein.

Anhand des Diagramms nach der Figur 8 wird das erfindungsgemäße Verfahren noch weiter erläutert. Zu erkennen sind Kurven für die unterschiedlichen Durchflussraten F beispielhaft für die Stoffe A1 (erster Ausgangsstoff), E1 (erster Eingangsstoff) sowie E2 (zweiter Eingangsstoff). Dabei stellt die fett gezeichnete Linie stets den relevanten entsprechenden Sollwert F_{A1,..,soll}, F_{E1,...,soll} bzw. F_{E2,...,soll} dar. Sofern der jeweilige Istwert F_{A1},ᵢₛₜ, F_{E1,ist} bzw. F_{E2},ᵢₛₜ gut eingeregelt ist, überdeckt sich der Istwert mit dem jeweiligen Sollwert und ist in Figur 8 nicht explizit zu erkennen. Sollte allerdings eine Abweichung zwischen dem Istwert und dem zugehörigen Sollwert vorliegen, so sind in Figur 8 die Kurven der jeweiligem Istwerte durch dünne Linien dargestellt, die mit den Bezugszeichen 21 - 30 versehen sind.

Es sind in Figur 8 unterschiedliche Zeitbereiche dargestellt, nämlich der Zeitbereich I (t < t₁), der Zeitbereich II (t₁ < t < t₂), der Zeitbereich III (t₂ < t < t3) sowie der Zeitbereich IV (t > t₃).

Zum Zeitbereich I arbeitet das Verfahren in einem stationären Betrieb, wie es in der Figur 6 (oder auch der Figur 3) dargestellt ist. Die Nutzervorgabe NV entspricht dem Sollwert F_{A1,soll}, also der gewünschten Durchflussrate für den ersten Ausgangsstoff A1. Unter Berücksichtigung der stöchiometrischen Verhältnisse wird ein Sollwert F_{E1,soll,R} für die Durchflussrate des ersten Eingangsstoffs E1, der Eingang in den entsprechenden Regler findet, vorgegeben. Der Sollwert F_{E2,soll,R} für die Durchflussrate des zweiten Eingangstoffs E2 ist abhängig von dem Istwert F_{E1,ist} der Durchflussrate des ersten Eingangsstoffes E1. So ist im Zeitbereich I beispielhaft mit dem Bezugszeichen 21 eine Schwankung des Istwerts F_{E1,ist} der Durchflussrate des ersten Eingangsstoffs E1 dargestellt. Der Sollwert F_{E2,soll,R} für die Durchflussrate des zweiten Eingangsstoffs orientiert sich an dem Istwert F_{E1,ist} der Durchflussrate des ersten Eingangsstoffs E1, was mit der entsprechenden gleichförmigen Schwankung, die mit dem Bezugszeichen 22 versehen ist, verdeutlicht wird. Bei einem gut eingestellten Regler folgt dabei der Istwert F_{E2,ist} der Durchflussrate des zweiten Eingangsstoffs E2 dem entsprechenden Sollwert F_{E2,soll,R}, so dass sich die (nicht explizit erkennbare) Istkurve mit der (hier fett dargestellten) Sollkurve überdeckt.

Zum Zeitpunkt t₁ wird nun anhand der Nutzervorgabe NV der Sollwert für die gewünschte Durchflussrate F_{A1,soll} schlagartig erhöht, beispielsweise um 50%. Die Regler werden hierfür außer Kraft gesetzt, wie in Figur 4 dargestellt. Vielmehr werden unmittelbar die temporären Stellgrößen S_{E1,temp} und S_{E2,temp} (siehe Figur 4) an die Stellglieder V_{E1}, V_{E2} ausgegeben. Zugleich werden durch die Steuerungseinheit SE die temporären Sollwerte F_{E1,soll,temp}, sowie F_{E2,soll,temp}, den Reglern zugrunde gelegt, wodurch sich der stufenförmige Verlauf in der Sollwertkurven zum Zeitpunkt t₁ in Figur 8 ergibt.

Nun reagieren die unterschiedlichen Regelstrecken der einzelnen Stoffe unterschiedlich auf die entsprechend geänderte schlagartige Sollwertvorgabe bzw. Stellgrößenänderung. Gemein ist allen drei Istwerten im Zeitbereich II, dass sie nicht konkret an einem Sollwert eingeregelt werden, sondern nur angesteuert werden, aufgrund von Erfahrungswerten, die in der Datenbasis DB (Figur 7) abgelegt sind.

Insofern verwundert es nicht, dass zum Zeitpunkt t₂ die Durchflussraten F_{A1,ist}, F_{E1,ist}, F_{E2,ist} nicht exakt auf den zugehörigen Sollwert eingeregelt sind. Allerdings ist zu erkennen, dass aufgrund der erfindungsgemäßen Ansteuerung die Istwerte der Durchflussraten bereits zum Zeitpunkt t₁' (wenige Sekunden nach t₁) recht nahe an den Sollwerten liegen und sich ein stationärer Zustand eingestellt hat. Zum Zeitpunkt t₂ erfolgt nun das Umschalten von den temporären Stellgrößen S_{E1,temp}, S_{E2,temp} auf die von den Reglern vorgegebenen Stellgrößen S_{E1,R}, S_{E2,R}, wie in Figur 5 dargestellt ist.

Dargestellt in Figur 8 ist ein Toleranzband 30 (schraffierte Fläche), dessen Breite dem Doppelten eines Toleranzwerts T entspricht. Damit ein Umschalten von den temporären Stellgrößen S_{E1,temp}, S_{E2,temp} auf die von den Reglern R vorgegebenen Stellgrößen S_{E1,R}, S_{E2,R}, erfolgen kann, müssen die Istwerte F_{A1,ist}, F_{E1,ist}, F_{E2},ᵢₛₜ der jeweiligen Durchflussraten innerhalb des Toleranzbandes 30 liegen; der Istwert darf folglich nicht mehr als der Toleranzwert T von einem Sollwert, im vorliegenden Fall der temporäre Sollwert F_{E2,soll,temp}, abweichen. Dies gilt auch für andere der zu regelnden Durchflussmengen; in der Figur 8 wird dies nur beispielhaft für den Stoff E2 illustriert. Grundsätzlich kann das Toleranzband außermittig positioniert sein; die Toleranzwerte nach oben und nach unten können sich voneinander unterscheiden.

Alternativ oder in Kombination kann die Einschwingphase zumindest eine vorgegebene Dauer aufweisen. Diese vorgegebene Dauer kann in Versuchsreihen ermittelt worden sein und hinterlegt werden. Sollte innerhalb dieser Dauer der Istwert nicht innerhalb des Toleranzbandes 30 liegen, wird die Produktionsanlage verriegelt, d.h. angehalten, da dann von einer Störung auszugehen ist.

Den Reglern werden in der Phase III nach wie vor die Sollwerte F_{E1,soll,temp}, F_{E2,soll,temp} zugrunde gelegt, die durch die Steuerungseinheit SE vorgegeben sind. Sehr schnell regeln sich nun die Istwerte für die Eingangsstoffe E1 und E2 auf die Sollwerte F_{E1,soll,temp}, F_{E2,soll,temp} ein, so dass es kurz nach dem Zeitpunkt t₂ zu einer Überdeckung der Sollwertkurven mit den Istwertkurven kommt.

Allerdings werden die Sollwerte nach wie vor weiter von der Steuerungseinheit SE vorgegeben, so dass die einzelnen Regelkreise noch nicht auf Schwankungen der Durchflussraten anderer Stoffe reagieren können. Sollte beispielsweise der Istwert F_{E1,ist} der Durchflussrate des ersten Eingangsstoffs E1 eine Schwankung aufweisen (Kurve 27), so bleibt dies ohne Auswirkung sowohl auf die Sollwertkurve als auch auf die Istwertkurve der Durchflussrate des zweiten Eingangsstoffs E2. In der Zeitspanne II, III, also zwischen t₁ und t₃, können sich die Regler einschwingen. Insofern wird die Zeit zwischen t₁ und t₃ als Einschwingphase bezeichnet.

Zum Zeitpunkt t₃ wird nun auch der Sollwert umgestellt, der dem jeweiligen Regler zugrunde gelegt wird. Nunmehr werden die temporären Sollwerte F_{E1,soll,temp}, F_{E2, soll,temp}, die von der Steuerungseinheit SE vorgegeben werden, durch die Sollwerte F_{E1,soll,R}, F_{E2, soll,R} ersetzt, die durch das Verfahren beeinflusst werden. Eine Schwankung des Istwerts F_{E1,ist} der Durchflussrate des ersten Eingangsstoffs E1 im Zeitbereich IV (Kurve 28) wirkt sich somit analog zum Zeitbereich I unmittelbar auf den Sollwert F_{E2,soll,R} der Durchflussrate des zweiten Eingangsstoffs E2 (Kurve 29) aus. Durch das Umschalten ergeben sich zum Zeitpunkt t₃ jeweils kleine Stufen in den Sollwertkurven. Die Istwerte werden aufgrund des zu diesem Zeitpunkt (t₃) eingeschwungenen Regelkreises schnell auf die neuen Sollwerte eingeregelt.

Die beispielhaft für die Durchflussraten der der Stoffe E1 und E2 gemachten Angaben können analog auch für sämtliche der anderen relevanten Stoffe und Durchflussraten angewendet werden.

In Figur 9 wird eine weitere Form des erfindungsgemäßen Verfahrens beschrieben. Für die Einschwingphase im Zeitbereich II sind nun für die Eingangsstoffe E1 und E2 die vom Prozess vorgegebenen Sollwerte F_{E1,soll,R} und F_{E2,soll,R} dargestellt. Diese orientieren sich an anderen im Verfahren gemessenen Istwerten von Durchflussraten. Da die Istwerte anderer Stoffe durch das erfindungsgemäße Vorgehen recht schnell einen stationären Zustand einnehmen, folgen die Sollwerte diesen Istwerten recht schnell in einen stationären Zustand.

Es ist zu erkennen, dass die Istwerte F_{E1,ist}, F_{E2,ist} kurz nach Beginn des Einschwingvorgangs t₁ bereits näher an den Sollwerten liegen können als die Sollwerte F_{E1,soll,R}, F_{E2,soll,R}, die der Prozess den Reglern vorgibt. Insofern wird deutlich schneller ein stationärer Zustand recht nahe am endgültigen stationären Zustand (IV) erreicht, was das rasche Einschwingen der Regler begünstigt. Dies gilt im Übrigen auch für das nach Figur 8 beschriebene Verfahren. Dort sind die vom Prozess vorgegebenen Sollwerte F_{E1,soll,R}, F_{E2,soll,R} in den Zeitbereichen II und III aus Gründen der Übersichtlichkeit nicht dargestellt.

Beim alternativen Verfahren entsprechend Figur 9 wird nun der Schritt nach Figur 5 (Zeitbereich III nach Figur 8) vollständig weggelassen. Vielmehr werden die Stellglieder derart lange mit der temporären Stellgröße beaufschlagt, bis sich sämtliche Regelkreise eingeschwungen haben. Dies ist nun daran zu erkennen, dass die Sollwerte F_{E1,soll,R} und F_{E2,soll,R} asymptotisch einem Grenzwert angenähert haben. Dies ist zum Zeitpunkt t₃ in jedem Fall gegeben. Dann werden die den Stellgliedern zugeführten Stellgrößen auf einmal umgeschaltet auf die von den Regler vorgegebenen Stellgrößen S_{E1,R} und S_{E1,R}. Die Dauer (t₃-t₁) der Einschwingphase II wird durch einen hinterlegten Wert vorgegeben.

Weiter oben wurde bereits die Überwachung des Prozesses anhand von Grenzwerten beschrieben; sofern ein Verlassen der zulässigen Grenzwerte festgestellt wird, wird die Produktionsanlage verriegelt. Im Rahmen des vorliegenden Verfahrens können die Grenzwerte in unterschiedlichen Zeitbereichen variieren. In den Zeitbereichen I und IV, in denen die Anlage im stationären Betrieb ist, werden recht enge Grenzwerte angewendet werden. In den Zeitbereichen II und III (der Einschwingphase) werden großzügigere Grenzwerte angewendet, d.h. größere Abweichungen werden toleriert.

Ein wesentlicher Vorteil des erfindungsgemäßen Verfahrens ist es, dass der zur neuen gewünschten Produktionsmenge gehörende stationäre Zustand der Anlage wesentlich schneller erreicht werden kann. Damit wird der Zeitraum, in denen diese großzügigeren Grenzwerte zugelassen werden müssen, deutlich verkürzt. Insgesamt ist das erfindungsgemäße Verfahren sicherer und es produziert weniger Ausschuss.

Durch das erfindungsgemäße Verfahren wird es auch einfacher, die Produktionsanlage bei Bedarf abzuschalten und wieder anzuschalten. Daher ist es komfortabler, die Anlage zu Wartungszwecken abzuschalten. Die Wartungsintervalle können so unproblematisch verkürzt werden, was die Zuverlässigkeit der Anlage erhöht. Zudem kann das Steuerungsverfahren um automatische Spülzyklen ergänzt werden, wodurch die Rohrleitungen automatisch frei von Nitroaromaten gespült werden was die Sicherheit der Anlage weiter verbessert

Zur Kontrolle, ob das Verfahren korrekt abläuft, kann eine Anzeige vorgesehen sein, aus der eine Bedienperson ein derzeitig resultierendes stöchiometrisches Verhältnis z.B. der ersten chemischen Reaktion C1 entnehmen kann. Beispielsweise beträgt der stöchiometrische Koeffizient der ersten chemischen Reaktion C1 zwischen den beiden Eingangstoffen E1 und E2 ν=2. Nun kann anhand der Istwerte der Durchflussmessungen der beiden Eingangsstoffe E1 und E2 unter Berücksichtigung der Konzentrationen dieser Stoffe berechnet werden, wie das tatsächliche stöchiometrische Verhältnis ist. Wird ein Wert 1,9 angezeigt, wird zuwenig vom Stoff E1 zugeführt, beim Wert 2,1 wird zu viel des Stoffs E1 zugeführt. Ein gewisse Abweichung vom stöchiometrischen Koeffizienten ν=2 kann beabsichtigt sein, wenn sichergestellt werden soll, dass einer der Stoffe umgesetzt wird. Dies kann auch durch die Recheneinheit 11 und/oder die stöchiometrische Funktion 12 bei der Berechnung von Sollwerten berücksichtigt werden.

In den Figuren 8 und 9 sind die Kurven der Sollwerte der Durchflussrate F_{E1,R} für den Stoff E1 mit dem eingeklammerten Bezugszeichen (NV) versehen. Damit soll ausgedrückt werden, dass sich insbesondere auch die gewünschte Durchflussrate des zugeführten Toluols E1 als Nutzervorgabe eignet.

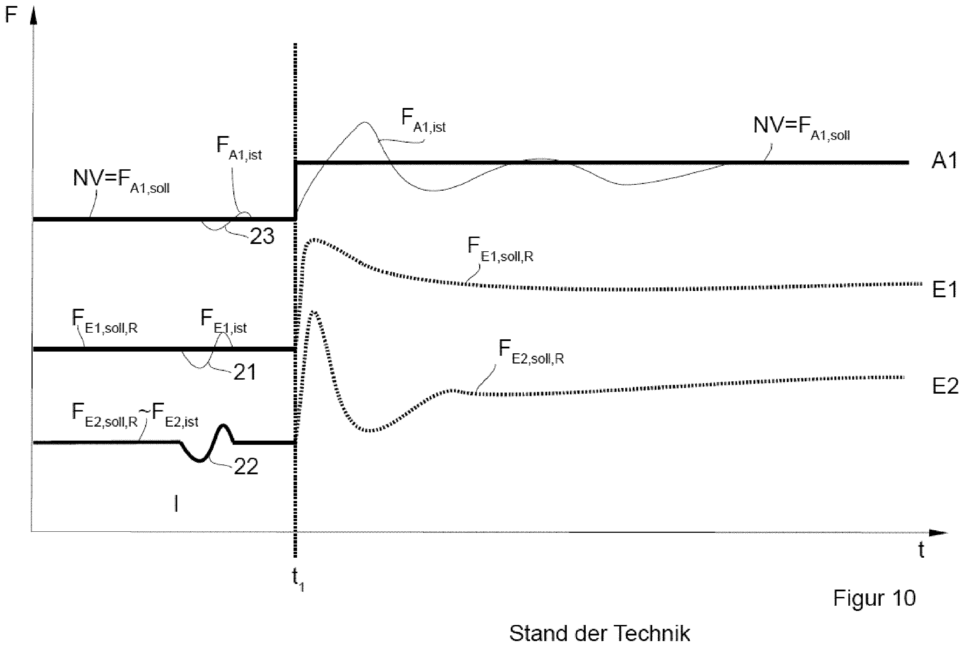

Zum Vergleich zeigt Figur 10 ein Verfahren ohne die erfindungsgemäße Ansteuerung. Zum Zeitpunkt t₁ erfolgt die stufenförmige Änderung der Nutzervorgabe NV. Hieran orientiert sich (auch unter Berücksichtigung der stöchiometrischen Bedingungen) auch der Sollwert F_{E2,soll,R} der Durchflussrate zumindest des einen anderen Stoffes E2, welcher dessen Regler zugrunde gelegt wird. Dieser bildet ein überschwingendes Verhalten aus. Aufgrund der hohen Schwankung im Eingang dieses Reglers, wird dieser Regler selbst instabil werden. Entsprechend deutlich mehr schwanken wird der Regler des Stoffes E1, der wiederum abhängig ist vom Istwert F_{E1,ist} der Durchflussrate des Stoffes E1. Die Ausstoßrate F_{A1,ist} des Endprodukts A1 wird lange Zeit instationär sein und sich sehr langsam an den Vorgabewert NV angleichen. Während dieser Zeit ist die Gefahr von Grenzwertüberschreitungen sehr hoch, so dass eine Notabschaltung wahrscheinlich ist.

Um dieser Problematik bislang zu entgegen, wurde die Produktionsrate F_{A1,soll} in kleinen Schritten erhöht. Die Schritte wurden dabei derart klein (Erhöhung der Produktionsrate um wenige Prozentpunke pro Schritt) gewählt, dass die einzelnen Regelkreise diesen Änderungen folgen konnten, ohne das es zu unerwünscht starken Schwankungen der Zusammensetzung des Reaktionsgemisches kam. Dies erforderte viel Zeit und erfahrenes Bedienpersonal. Ferner erfüllen die Ausgangsstoffe während dieses Hochfahrens keine hohen Anforderungen an Reinheit; während des Hochfahrens wurde folglich viel Ausschuss produziert.

### Bezugszeichenliste

- E1, E2: Eingangsstoff
- C1, C2: chemische Reaktionen
- A1, A2: Ausgangsstoff
- Z1, Z2: Zwischenstoff
- W1, W2, W3: weiterer Stoff
- Index i: allgemeiner Index für die Stoffe oder Reaktionen
- Index ist: Index Istwerte
- Index soll: Index Sollwerte
- Index temp: Index für temporär vorgegebene Werte
- Index R: Index für vom Regelkreis erzeugte Werte

- F: Durchflussrate
- R: Regler
- S: Stellgröße
- V: Stellglied
- SE: Steuerungseinheit
- DB: Datenbasis
- NV: Nutzervorgabe
- ν: stöchiometrischer Koeffizient
- t: Zeit
- T: Toleranzwert

- 11: Recheneinheit
- 12: stöchiometrische Funktion
- 13: Steuerungsfunktion
- 14: mathematische Zuordnung

- 21-29: Istwert-Kurven, die von Sollwertkurven abweichen
- 30: Toleranzband

## Patentansprüche

1. Verfahren zur kontinuierlichen Herstellung eines Produkts (A1) über zumindest zwei miteinander gekoppelte chemische Reaktionen (C1, C2),
wobei zumindest zwei Eingangsstoffe (E1, E2) einer ersten chemischen Reaktion (C1) zugeführt werden, wobei durch die erste chemische Reaktion (C1) aus den Eingangsstoffen (E1, E2) eine Mehrzahl von Zwischenstoffen (Z1, Z2) erzeugt werden,
wobei zumindest einer der Zwischenstoffe (Z2) einer zweiten chemischen Reaktion (C2) zugeführt wird, wobei durch die zweite chemische Reaktion (C2) der zumindest eine zugeführte Zwischenstoff (Z2), insbesondere unter Verwendung zumindest eines weiteren Stoffs (W1, W2) in einer zweiten chemischen Reaktion (C2) zu einer Mehrzahl von Ausgangsstoff (A1, A2) weiterverarbeitet wird, nämlich zum chemischen Produkt (A1) und zumindest einem weiteren Ausgangsstoff (A2), wobei die Durchflussraten (Fᵢ) der zugeführten Stoffe (E1, E2, Z1, W1, W2, A2), die einer der Reaktionen (C1, C2) zugeführt werden, durch ein jeweiliges Stellglied (V_{E1}, V_{E2}, V_{W1}, V_{W2}, V_{Z2}, V_{A1}) eingestellt werden, wobei jedem der zugeführten Stoffe ein separates Stellglied zugeordnet ist, wobei zumindest eines der Stellglieder mit jeweils einer von einem Regler (R_{E2}, Rᵢ) vorgegebenen Stellgröße (S_{E2,R}, S_{i,R}) beaufschlagt wird,
**dadurch gekennzeichnet,**
**dass** für eine Änderung der Produktionsrate des chemischen Produkts (A1) zumindest eines dieser Stellglieder (V_{E2}, Vᵢ) während einer Einschwingphase (II,III) anstelle der von den jeweiligen Reglern (R_{E2}, Ri) vorgegebenen Stellgrößen (S_{E2,R}, S_{i,R}) jeweils mit einer temporären Stellgröße (S_{E2,temp}, S_{i,temp}) beaufschlagt wird, wobei die temporäre Stellgröße (S_{E2,temp}, S_{i,temp}) bzw. die temporären Stellgrößen in Abhängigkeit eines Vorgabewertes (NV) von zumindest einer Steuerungseinheit (SE) erzeugt werden.

2. Verfahren nach Anspruch 1,
**dadurch gekennzeichnet,**
**dass** ein Stellglied (V_{E2}, Vᵢ) während der Einschwingphase (II, II-III) mit einer solchen temporären Stellgröße (S_{E1,temp}, S_{i,temp}) beaufschlagt wird, wobei mit diesem Stellglied (V_{E1}, Vᵢ) die Durchflussrate eines Stoffes (E1) eingestellt wird, der einer der chemischen Reaktionen (C1) zugeführt wird und wobei insbesondere die Nutzervorgabe (NV) den Sollwert (F_{E1,soll,temp}, Fi_{,soll,temp}) des Durchflusses dieses Stoffes (E1) darstellt.

3. Verfahren nach einem der vorherigen Ansprüche,
**dadurch gekennzeichnet,**
**dass** sämtliche Stellglieder (V_{E1}, V_{E2}, Vᵢ) während einer Einschwingphase durch jeweils eine temporäre Stellgröße (S_{E1,temp}, S_{E2,temp}, S_{i,temp}) beaufschlagt werden.

4. Verfahren nach einem der vorherigen Ansprüche,
**dadurch gekennzeichnet,**
**dass** die temporäre Stellgröße (S_{E2,temp}, S_{i,temp}) bzw. die temporären Stellgrößen durch zumindest eine Steuerungsfunktion (13) der Steuerungseinheit (SE) mithilfe einer Datenbasis (DB) erzeugt wird bzw. werden, in welcher mathematische Zuordnungen (14) zwischen einer temporären Durchflussrate (F_{E2,soll,temp}, F_{i,soll,temp}) und einer dazugehörenden Stellgröße (S_{E2,temp}, S_{i,temp}) für jede dieser Stellglieder (V_{E2}, Vᵢ) hinterlegt sind.

5. Verfahren nach einem der vorherigen Ansprüche,
**dadurch gekennzeichnet,**
**dass** für zumindest einen zugeführten Stoff (E1, E2, Z1, W1, W2, A2) ein temporärer Sollwert (F_{E2,soll,temp}, F_{i,soll,temp}) einer temporären Durchflussrate unter Berücksichtigung des Vorgabewertes (NV), anhand einer, insbesondere stöchiometrischen, Funktion (12) erzeugt wird, wobei dieser temporäre Sollwert (F_{E2,soll,temp,} F_{i,soll,temp}) der Erzeugung der zugehörigen temporären Stellgröße (S_{E2,temp}, S_{i,temp}) zugrunde gelegt wird.

6. Verfahren nach einem der vorherigen Ansprüche,
**dadurch gekennzeichnet,**
**dass** nach Ablauf (t₃) einer Einschwingphase (II;II-III) umgeschaltet wird von einer Beaufschlagung der Stellglieder (V_{E2}, Vᵢ) mit den temporären Stellgrößen (S_{E2,temp}, S_{i,temp}) auf eine Beaufschlagung mit den von den jeweiligen Reglern (R_{E2}, Rᵢ) vorgegebenen Stellgrößen (S_{E2,R}, S_{i,R}).

7. Verfahren nach dem vorherigen Anspruch,
**dadurch gekennzeichnet,**
**dass** der Ablauf der Einschwingphase (t₃) frühestens dann erreicht ist, wenn sämtliche Regler (Rᵢ) eine Stellgröße (F_{i,soll,R}), ausgeben, deren Schwankungsbreite unterhalb eines vorgegeben Schwellwertes liegen.

8. Verfahren nach einem der vorherigen Ansprüche, insbesondere nach den Ansprüchen 5 und 6 oder den Ansprüchen 5 bis 7,
**dadurch gekennzeichnet,**
**dass** der Ablauf der Einschwingphase (t₃) frühestens dann erreicht ist, wenn eine Abweichung des Istwerts (F_{E2,ist}, F_{i,ist}) der Durchflussrate des Stoffes, welche durch das mit der temporären Stellgröße (S_{E2,temp}, S_{i,temp}) beaufschlagten Stellglied (V_{E2}, Vᵢ) eingestellt wird, von einem zugehörigen Sollwert, insbesondere dem temporären Sollwert (F_{E1,soll,temp}, F_{E2,soll,temp}, F_{i,soll,temp}), geringer als ein vorgegebener Toleranzwert (T) ist.

9. Verfahren nach einem der Ansprüche 5 bis 8,
**dadurch gekennzeichnet,**
**dass** nach Ablauf (t₃) einer ersten Einschwingphase (II) umgeschaltet wird auf eine zweite Einschwingphase (III), während der das zumindest eine Stellglied (S_{E2}, Sᵢ) mit einer von dem jeweiligen Regler (R_{E2}, Rᵢ) vorgegebenen Stellgrößen (S_{E2,R}, S_{i,R}) beaufschlagt wird, wobei dem Regler (R_{E2,} Rᵢ) ein temporärer Sollwert (F_{E2,soll,temp}, F_{i,soll,temp}) zugrunde gelegt wird, der von der Steuerungseinheit (SE) vorgegeben wird.

10. Verfahren nach einem der vorherigen Ansprüche,
**dadurch gekennzeichnet,**
**dass** die Dauer der Einschwingphase (t₃-t₁), der ersten Einschwingphase (t₂-t₁) und/oder der zweiten Einschwingphase (t₃-t₂), durch einen statisch hinterlegten Wert definiert wird oder anhand einer statisch hinterlegten mathematischen Zuordnung ermittelt wird.

11. Verfahren nach einem der vorherigen Ansprüche, wobei das Verfahren zur Herstellung von Dinitrotoluol (A1) eingesetzt wird,
**dadurch gekennzeichnet,**
**dass**
- der erste Eingangsstoff (E1) Toluol,
- der zweite Eingangsstoff (E2) Salpetersäure,
- der erste Zwischenstoff (Z1) eine Absäure,
- der zweite Zwischenstoff (Z2) Mononitrotoluol,
- der erste Ausgangsstoff (A1) Dinitrotoluol,
- der zweite Ausgangsstoff (A2) Schwefelsäure,
- der erste weitere Stoff (W1) Schwefelsäure, und
- der zweite weitere Stoff (W2) Salpetersäure
ist,
wobei die erste chemische Reaktion (C1) eine Nitrierung von Toluol (E1), Salpetersäure (E2) und Schwefelsäure (A2) zu Mononitrotoluol (Z2) umfasst, und wobei die zweite chemische Reaktion eine Nitrierung von Mononitrotoluol (Z2) mit Salpetersäure (W2) und Schwefelsäure (W1) zu Dinitrotoluol (A1) umfasst.

12. Verwendung einer chemischen Produktionsanlage zur Durchführung des Verfahrens nach einem der vorherigen Ansprüche.

13. Regelungs- und Steuerungsanordnung, die derart eingerichtet ist, das Verfahren nach einem der Ansprüche 1 bis 11 zu regeln und zu steuern, umfassend mehrere Stellglieder (Vᵢ) zur Beeinflussung der Durchflussrate (Fᵢ) eines fließenden Stoffes (E1, E2, Z1, W1, W2, A2),
eine Steuerungseinheit (SE) sowie mehrere Regler (Ri) zur wechselnden Beaufschlagung der Stellglieder (Vᵢ) mit einer Stellgröße (S_{i,temp}, S_{i,R}),
eine Datenbasis (DB) zur Hinterlegung von Zuordnungen (14), anhand derer die Steuerungseinheit temporäre Stellgrößen (S_{i,temp}) in Abhängigkeit von Sollwerten (F_{i,soll,temp}) ausgibt.

## Claims

1. Process for the continuous production of a product (A1) via at least two coupled chemical reactions (C1, C2),
where at least two starting materials (E1, E2) are fed to a first chemical reaction (C1) and a plurality of intermediates (Z1, Z2) are produced from the starting materials (E1, E2) by the first chemical reaction (C1), where at least one of the intermediates (Z2) is fed to a second chemical reaction (C2) and the at least one introduced intermediate (Z2) is processed further by the second chemical reaction (C2), in particular using at least one further material (W1, W2) in a second chemical reaction (C2) to give a plurality of output materials (A1, A2), namely to form the chemical product (A1) and at least one further output material (A2), where the flow rates (Fi) of the introduced materials (E1, E2, Z1, W1, W2, A2) which are fed to one of the reactions (C1, C2) are set by a respective regulating device (V_{E1}, V_{E2}, V_{W1}, V_{W2}, V_{Z2}, V_{A1}), with a separate regulating device being assigned to each of the materials fed in and at least one of the regulating devices being supplied in each case with a control variable (S_{E2,R,} S_{i,R}) prescribed by a regulator (R_{E2,} Rᵢ),
**characterized**
**in that**, to change the production rate of the chemical product (A1), at least one of these regulating devices (V_{E2}, Vᵢ) is supplied during a settling-down phase (II, III) in each case with a temporary control variable (S_{E2,temp}, S_{i,temp}) instead of the control variables (S_{E2,R}, S_{i,R}) prescribed by the respective regulators (R_{E2,} Rᵢ), where the temporary control variable (S_{E2,}tₑₘₚ, S_{i,temp}) or the temporary control variables are generated as a function of a prescribed value (NV) from at least one control unit (SE).

2. Process according to Claim 1,
**characterized in that**
a regulating device (V_{E2}, Vᵢ) is supplied during the settling-down phase (II, II-III) with such a temporary control variable (S_{E1,temp}, S_{i,temp}), where the flow rate of a material (E1) which is fed to one of the chemical reactions (C1) is set by means of this regulating device (V_{E1}, Vᵢ) and wherein, in particular, the user setting (NV) represents the intended value (F_{E1,int,temp}, Fi_{,int,temp}) of the flow of this material (E1).

3. Process according to either of the preceding claims,
**characterized in that**
all regulating devices (V_{E1}, V_{E2}, Vᵢ) are supplied in each case with a temporary control variable (S_{E1,temp}, S_{E2,temp}, S_{i,temp}) during a settling-down phase.

4. Process according to any of the preceding claims,
**characterized in that**
the temporary control variable (S_{E2,temp}, S_{i,temp}) or the temporary control variables is/are generated by means of at least one control function (13) of the control unit (SE) with the aid of a database (DB), in which mathematical relationships (14) between a temporary flow rate (F_{E2,int,temp}, F_{i,int,temp}) and an associated control variable (S_{E2,temp}, S_{i,temp}) for each of these regulating devices (V_{E2}, Vᵢ) are stored.

5. Process according to any of the preceding claims,
**characterized in that**
a temporary intended value (F_{E2,int,temp}, Fi_{,int,temp}) of a temporary flow rate is generated, taking into account the prescribed value (NV), for at least one material fed in (E1, E2, Z1, W1, W2, A2) with the aid of a, in particular stoichiometric, function (12), and this temporary intended value (F_{E2,int,temp}, F_{i,int,temp}) is used as a basis for generating the associated temporary control variable (S_{E2,temp}, S_{i,temp}) .

6. Process according to any of the preceding claims,
**characterized in that**,
after a settling-down phase (II;II-III) has concluded (t₃), a change is made from supplying the regulating devices (V_{E2}, Vᵢ) with the temporary control variables (S_{E2,temp}, S_{i,temp}) to supplying them with the control variables (S_{E2,R}, S_{i,R}) prescribed by the respective regulators (R_{E2,} Rᵢ).

7. Process according to the preceding claim,
**characterized in that**
the conclusion of the settling-down phase (t₃) is reached at the earliest when all regulators (Rᵢ) supply a control variable (F_{i,int,R}) whose range of fluctuation is below a prescribed threshold value.

8. Process according to any of the preceding claims, in particular according to Claims 5 and 6 or Claims 5 to 7,
**characterized in that**
the conclusion of the settling-down phase (t₃) is reached at the earliest when a deviation of the actual value (F_{E2,act}, F_{i,act}) of the flow rate of the material which is set by means of the regulating device (V_{E2}, Vᵢ) supplied with the temporary control variable (S_{E2,temp}, S_{i,temp}) from an associated intended value, in particular the temporary intended value (F_{E1,int,temp}, F_{E2}, int,temp, F_{i,int,temp}), is smaller than a prescribed tolerance value (T) .

9. Process according to any of Claims 5 to 8,
**characterized in that**,
after a first settling-down phase (II) has concluded (t₃), a change is made to a second settling-down phase (III) during which the at least one regulating device (S_{E2}, Sᵢ) is supplied with a control variable (S_{E2,R}, S_{i,R}) prescribed by the respective regulator (R_{E2,} Rᵢ), where a temporary intended value (F_{E2,int,temp}, Fi_{,int,temp}) which is prescribed by the control unit (SE) is used as a basis for the regulator (R_{E2,} Rᵢ).

10. Process according to any of the preceding claims,
**characterized in that**
the duration of the settling-down phase (t₃-t₁) of the first settling-down phase (t₂-t₁) and/or of the second settling-down phase (t₃-t₂) is defined by a statically stored value or is determined with the aid of a statically stored mathematical relationship.

11. Process according to any of the preceding claims, where the process is used for producing dinitrotoluene (A1),
**characterized in that**
- the first starting material (E1) is toluene,
- the second starting material (E2) is nitric acid,
- the first intermediate (Z1) is a used acid,
- the second intermediate (Z2) is mononitrotoluene,
- the first output material (A1) is dinitrotoluene,
- the second output material (A2) is sulfuric acid,
- the first further material (W1) is sulfuric acid and
- the second material (W2) is nitric acid,
where the first chemical reaction (C1) comprises a nitration of toluene (E1), nitric acid (E2) and sulfuric acid (A2) to form mononitrotoluene (Z2) and the second chemical reaction comprises a nitration of mononitrotoluene (Z2) by means of nitric acid (W2) and sulfuric acid (W1) to form dinitrotoluene (A1).

12. Use of a chemical production plant for carrying out the process according to any of the preceding claims.

13. Regulating and control arrangement which is configured for regulating and controlling the process according to any of Claims 1 to 11, comprising a plurality of regulating devices (Vᵢ) for influencing the flow rate (Fᵢ) of a flowing material (E1, E2, Z1, W1, W2, A2),
a control unit (SE) and a plurality of regulators (Ri) for variably supplying the regulating devices (Vᵢ) with a control variable (S_{i,temp}, S_{i,R}),
a database (DB) for storing relationships (14) with the aid of which the control unit supplies temporary control variables (S_{i,temp}) as a function of intended values (F_{i,int,temp}).

## Revendications

1. Procédé de fabrication continue d'un produit (A1) par au moins deux réactions chimiques (C1, C2) couplées l'une avec l'autre,
au moins deux matières d'entrée (E1, E2) étant introduites dans une première réaction chimique (C1), une pluralité de matières intermédiaires (Z1, Z2) étant formées par la première réaction chimique (C1) à partir des matières d'entrée (E1, E2),
au moins une des matières intermédiaires (Z2) étant introduite dans une deuxième réaction chimique (C2), ladite au moins une matière intermédiaire introduite (Z2) étant transformée par la deuxième réaction chimique (C2), notamment en utilisant au moins une matière supplémentaire (W1, W2) dans une deuxième réaction chimique (C2), en une pluralité de matières de sortie (A1, A2), à savoir en le produit chimique (A1) et au moins une autre matière de sortie (A2), les débits (Fᵢ) des matières introduites (E1, E2, Z1, W1, W2, A2), qui sont introduites dans une des réactions (C1, C2), étant ajustés par un élément de réglage correspondant (V_{E1}, V_{E2}, V_{W1}, V_{W2}, V_{Z2}, V_{A1}), un élément de réglage séparé étant attribué à chacune des matières introduites, au moins un des éléments de réglage étant chargé avec une grandeur de réglage (S_{E2,R}, S_{i,R}) prescrite par un régulateur (R_{E2}, Rᵢ),
**caractérisé en ce que**
pour une modification du taux de production du produit chimique (A1), au moins un de ces éléments de réglage (V_{E2}, Vᵢ) est chargé pendant une phase de transition (II, III) avec une grandeur de réglage temporaire (S_{E2,temp}, S_{i,temp}) au lieu des grandeurs de réglages (S_{E2,R}, S_{i,R}) prescrites par les régulateurs respectifs (R₂, Rᵢ), la grandeur de réglage temporaire (S_{E2,temp}, S_{i,temp}) ou les grandeurs de réglage temporaires étant générées en fonction d'une valeur d'instruction (NV) d'au moins une unité de commande (SE).

2. Procédé selon la revendication 1, **caractérisé en ce qu'**un élément de réglage (V_{E2}, Vᵢ) est chargé pendant la phase de transition (II, II-III) avec une telle grandeur de réglage temporaire (S_{E1,temp}, S_{i,temp}), cet élément de réglage (V_{E1}, Vᵢ) ajustant le débit d'une matière (E1) qui est introduite dans une des réactions chimiques (C1) et l'instruction de l'utilisateur (NV) étant la valeur de consigne (F_{E1,consigne,temp}, F_{i,consigne,temp}) du débit de cette matière (E1).

3. Procédé selon l'une quelconque des revendications précédentes, **caractérisé en ce que** tous les éléments de réglage (V_{E1}, V_{E2}, Vᵢ) sont chargés pendant une phase de transition avec à chaque fois une grandeur de réglage temporaire (S_{E1,temp}, S_{E2,temp}, S_{i,temp}).

4. Procédé selon l'une quelconque des revendications précédentes, **caractérisé en ce que** la grandeur de réglage temporaire (S_{E2,temp}, S_{i,temp}) ou les grandeurs de réglage temporaires sont générées par au moins une fonction de commande (13) de l'unité de commande (SE) à l'aide d'une base de données (DB), dans laquelle des attributions mathématiques (14) entre un débit temporaire (F_{E2}, _{consigne,temp}, F_{i,consigne,temp}) et une grandeur de réglage correspondante (S_{E2,temp}, S_{i,temp}) sont déposées pour chacun de ces éléments de réglage (V_{E2}, Vᵢ).

5. Procédé selon l'une quelconque des revendications précédentes, **caractérisé en ce que** pour au moins une matière introduite (E1, E2, Z1, W1, W2, A2), une valeur de consigne temporaire (F_{E2,consigne,temp}, F_{i,consigne,temp}) d'un débit temporaire est générée en prenant en compte la valeur d'instruction (NV) à l'aide d'une fonction notamment stoechiométrique (12), cette valeur de consigne temporaire (F_{E2,consigne,temp}, F_{i,consigne,temp}) étant sous-jacente à la génération de la grandeur de réglage temporaire correspondante (S_{E2,temp}, S_{i,temp}).

6. Procédé selon l'une quelconque des revendications précédentes, **caractérisé en ce qu'**après l'écoulement (t₃) d'une phase de transition (II ; II-III), le chargement des éléments de réglage (V_{E2}, Vᵢ) avec les grandeurs de réglage temporaires (S_{E2,temp}, S_{i,temp}) est remplacé par un chargement avec les grandeurs de réglage (S_{E2,R}, S_{i,R}) prescrites par les régulateurs respectifs (R_{E2}, Rᵢ).

7. Procédé selon la revendication précédente, **caractérisé en ce que** l'écoulement de la phase de transition (t₃) est atteint au plus tôt lorsque tous les régulateurs (Rᵢ) émettent une grandeur de réglage (F_{i,consigne,R}) dont la marge de fluctuation est inférieure à une valeur de seuil prescrite.

8. Procédé selon l'une quelconque des revendications précédentes, notamment selon les revendications 5 et 6 ou les revendications 5 à 7, **caractérisé en ce que** l'écoulement de la phase de transition (t₃) est atteint au plus tôt lorsqu'une déviation de la valeur réelle (F_{E2,réelle}, F_{i,réelle}) du débit de la matière qui est ajusté avec l'élément de réglage (V_{E2}, Vᵢ) chargé avec la grandeur de réglage temporaire (S_{E2,temp}, S_{i,temp}), par rapport à une valeur de consigne correspondante, notamment la valeur de consigne temporaire (F_{E1,consigne,temp}, F_{E2,consigne,temp,} F_{i,consigne,temp}), est inférieure à une valeur de tolérance prescrite (T).

9. Procédé selon l'une quelconque des revendications 5 à 8, **caractérisé en ce qu'**après l'écoulement (t₃) d'une première phase de transition (II), une deuxième phase de transition (III) a lieu, pendant laquelle ledit au moins un élément de réglage (S_{E2}, Sᵢ) est chargé avec une grandeur de réglage (S_{E2,R}, S_{i,R}) prescrite par le régulateur respectif (R_{E2}, Rᵢ), une valeur de consigne temporaire (F_{E2,consigne,temp}, F_{i,consigne,temp}), qui est prescrite par l'unité de commande (SE), étant sous-jacente au régulateur (R_{E2}, Rᵢ).

10. Procédé selon l'une quelconque des revendications précédentes, **caractérisé en ce que** la durée de la phase de transition (t₃-t₁₎, de la première phase de transition (t₂-t₁) et/ou de la deuxième phase de transition (t₃-t₂) est définie par une valeur statique déposée ou est déterminée par une attribution mathématique statique déposée.

11. Procédé selon l'une quelconque des revendications précédentes, le procédé étant utilisé pour la fabrication de dinitrotoluène (A1), **caractérisé en ce que**
- la première matière d'entrée (E1) est le toluène,
- la deuxième matière d'entrée (E2) est l'acide nitrique,
- la première matière intermédiaire (Z1) est un acide résiduaire,
- la deuxième matière intermédiaire (Z2) est le mononitrotoluène,
- la première matière de sortie (A1) est le dinitrotoluène,
- la deuxième matière de sortie (A2) est l'acide sulfurique,
- la première matière supplémentaire (W1) est l'acide sulfurique et
- la deuxième matière supplémentaire (W2) est l'acide nitrique,
la première réaction chimique (C1) comprenant une nitration de toluène (E1), acide nitrique (E2) et acide sulfurique (A2) en mononitrotoluène (Z2), et la deuxième réaction chimique comprenant une nitration de mononitrotoluène (Z2) avec de l'acide nitrique (W2) et de l'acide sulfurique (W1) en dinitrotoluène (A1).

12. Utilisation d'une unité de production chimique pour la réalisation du procédé selon l'une quelconque des revendications précédentes.

13. Agencement de régulation et de commande, qui est conçu pour réguler et commander le procédé selon l'une quelconque des revendications 1 à 11, comprenant :
plusieurs éléments de réglage (Vᵢ) pour influencer le débit (Fᵢ) d'une matière en écoulement (E1, E2, Z1, W1, W2, A2),
une unité de commande (SE), ainsi que plusieurs régulateurs (Rᵢ), pour charger de manière variable les éléments de réglage (Vᵢ) avec une grandeur de réglage (S_{i,temp}, S_{i,R}),
une base de données (DB) pour déposer des attributions (14), à partir desquelles l'unité de commande émet des grandeurs de réglage temporaires (Sᵢ,ₜₑₘₚ) en fonction de valeurs de consigne (F_{i,consigne,temp}).
